# EUROPEAN PATENT SPECIFICATION

(11) **EP 2 056 834 B1**
(45) Date of publication and mention of the grant of the patent: **29.08.2012**
(21) Application number: 07841736.7
(22) Date of filing: 31.08.2007
(51) Int. Cl.: A61K 31/52, A61K 31/7076, A61K 49/00

(54) **METHODS AND COMPOSITIONS FOR INCREASING PATIENT TOLERABILITY DURING MYOCARDIAL IMAGING METHODS**
VERFAHREN UND ZUSAMMENSETZUNGEN FÜR ERHÖHTE PATIENTENVERTRÄGLICHKEIT WÄHREND MYOKARDIALER BILDGEBUNGSVERFAHREN
MÉTHODES ET COMPOSITIONS PERMETTANT D'AUGMENTER LA TOLÉRABILITÉ DE PATIENTS PENDANT DES MÉTHODES D'IMAGERIE MYOCARDIQUE

(30) Priority: 01.09.2006 US 841842 P
(43) Date of publication of application: 13.05.2009
(73) Proprietor: Gilead Sciences, Inc., Foster City, CA 94404 (US)
(72) Inventor: LIEU, Hsiao, D., Burlingame, CA 94010 (US); BLACKBURN, Brent, Los Altos, CA 94022 (US); BELARDINELLI, Luiz, Palo Alto, CA 94306 (US)
(74) Representative: Schnappauf, Georg
(86) International application number: PCT/US2007/077410
(87) International publication number: WO 2008/028140

(56) References cited:
- WO-A-01/62979
- US-A1- 2004 064 039
- HENDEL ET AL: "Initial Clinical Experience With Regadenoson, a Novel Selective A2A Agonist for Pharmacologic Stress Single-Photon Emission Computed Tomography Myocardial Perfusion Imaging" JOURNAL OF THE AMERICAN COLLEGE OF CARDIOLOGY, ELSEVIER, NEW YORK, NY, US, vol. 46, no. 11, 6 December 2005 (2005-12-06), pages 2069-2075, XP005191495 ISSN: 0735-1097
- GAO Z ET AL: "NOVEL SHORT-ACTING A2A ADENOSINE RECEPTOR AGONISTS FOR CORONARY VASODILATION: INVERSE RELATIONSHIP BETWEEN AFFINITY AND DURATION OFACTION OF A2A AGONISTS" JOURNAL OF PHARMACOLOGY AND EXPERIMENTAL THERAPEUTICS, AMERICAN SOCIETY FOR PHARMACOLOGY AND, US, vol. 298, no. 1, 2001, pages 209-218, XP001021027 ISSN: 0022-3565
- RIOU LAURENT M ET AL: "Influence of propranolol, enalaprilat, verapamil, and caffeine on adenosine A(2A)-receptor-mediated coronary vasodilation" JOURNAL OF THE AMERICAN COLLEGE OF CARDIOLOGY, ELSEVIER, NEW YORK, NY, US, vol. 40, no. 9, 6 November 2002 (2002-11-06), pages 1687-1694, XP009094876 ISSN: 0735-1097 cited in the application
- ZHAO G ET AL: "Effects of caffeine on coronary vasodilation and sinus tachycardia induced by Regadenoson, a novel adenosine A2A receptor agonist, in conscious dogs" EUROPEAN HEART JOURNAL, vol. 27, no. Suppl. 1, August 2006 (2006-08), page 424, XP009094877 & WORLD CONGRESS OF CARDIOLOGY; BARCELONA, SPAIN; SEPTEMBER 02 -06, 2006 ISSN: 0195-668X
- KOEPFLI P ET AL: "Interaction of caffeine with regadenoson-induced hyperemic myocardial blood flow as measured by PET" EUROPEAN HEART JOURNAL, vol. 27, no. Suppl. 1, August 2006 (2006-08), page 175, XP009094936 & WORLD CONGRESS OF CARDIOLOGY; BARCELONA, SPAIN; SEPTEMBER 02 -06, 2006 ISSN: 0195-668X
- MARTIN PAULINE L ET AL: "Pharmacology of 2-cyclohexylmethylidenehydrazinoadenosine (WRC-0470), a novel, short-acting adenosine A-2A receptor agonist that produces selective coronary vasodilation" DRUG DEVELOPMENT RESEARCH, vol. 40, no. 4, 1997, pages 313-324, XP002466020 ISSN: 0272-4391
- ZHAO GONG ET AL: "Caffeine attenuates the duration of coronary vasodilation and changes in hemodynamics induced by regadenoson (CVT-3146), a novel adenosine A2A receptor agonist" JOURNAL OF CARDIOVASCULAR PHARMACOLOGY, NEW YORK, NY, US, vol. 49, no. 6, June 2007 (2007-06), pages 369-375, XP009094871 ISSN: 0160-2446

## Description

### Field of the Invention

This invention relates to agents and compositions for increasing patient tolerability during myocardial imaging. According to the invention caffeine and one or more adenosine A_{2A} receptor agonists are administered to a mammal undergoing myocardial imaging.

### Description of the Art

Myocardial perfusion imaging (MPI) is a diagnostic technique useful for the detection and characterization of coronary artery disease. Perfusion imaging uses materials such as radionuclucides to identify areas of insufficient blood flow. In MPI, blood flow is measured at rest, and the result compared with the blood flow measured during exercise on a treadmill (cardiac stress testing), such exertion being necessary to stimulate blood flow. Unfortunately, many patients are unable to exercise at levels necessary to provide sufficient blood flow, due to medical conditions such as peripheral vascular disease, arthritis, and the like.

Therefore, pharmacological agents that increase CBF for a short period of time are of great benefit, particularly one that did not cause peripheral vasodilation. Several different types of vasodilators are currently known for use in perfusion imaging. Dipyridamole is one such effective vasodilator, but side effects such as pain and nausea limit the usefulness of treatment with this compound.

Another currently marketed vasodilator is AdenoScan ®( Astellas Pharma US, Inc.) which is a formulation of a naturally occurring adenosine. Adenosine, a naturally occurring nucleoside, exerts its biological effects by interacting with a family of adenosine receptors characterized as subtypes A₁, A_{2A}, A_{2B}, and A₃. Unfortunately, the use of adenosine is limited due to side effects such as flushing, chest discomfort, the urge to breathe deeply, headache, throat, neck, and jaw pain. These adverse effects of adenosine are due to the activation of other adenosine receptor subtypes in addition to A_{2A}, which mediates the vasodilatory effects of adenosine. Additionally, the short half-life of adenosine necessitates multiple treatments during the procedure, further limiting its use.

Other potent and selective agonists for the A_{2A} adenosine receptor are known. For example, MRE-0470 (Medco) is an adenosine A_{2A} receptor agonist that is a potent and selective derivative of adenosine. WRC-0470 (Medco) is an adenosine A_{2A} agonist used as an adjuvant in imaging. These compounds have a high affinity for the A_{2A} receptor, and, consequently, a long duration of action, which is undesirable in imaging.

Thus, there is still a need for a method of producing rapid and maximal coronary vasodilation in mammals without causing corresponding peripheral vasodilation, which would be useful for myocardial imaging with radionuclide agents. Preferred compounds would be selective for the A_{2A} adenosine receptor and have a short duration of action (although longer acting than compounds such as adenosine), thus obviating the need for multiple dosing.

### SUMMARY OF THE INVENTION

The following are aspects of this invention:

A pharmaceutical composition comprising at least 50 mg caffeine, at least 10 µg of at least one partial A_{2A} receptor agonist, and at least one pharmaceutical excipient, wherein the partial A_{2A} receptor agonist is selected from the group consisting of CVT-3033, Regadenoson, and combinations thereof.

Caffeine for use in a method of increasing patient tolerability during vasodilator induced myocardial stress perfusion imaging of a mammal, comprising administering a therapeutically effective amount of caffeine and at least 10 µg of at least one partial A_{2A} receptor agonist to the mammal, wherein the partial A_{2A} receptor agonist is selected from the group consisting of CVT-3033, Regadenoson, and combinations thereof.

The method of increasing patient tolerability during vasodilator induced myocardial stress perfusion imaging of a mammal, may comprise administering caffeine and no more than about 1000 µg of the partial A_{2A} receptor agonist to the mammal.

The method of increasing patient tolerability during vasodilator induced myocardial stress perfusion imaging of a mammal, may comprise administering caffeine and the partial A_{2A} receptor agonist in an amount ranging from about 10 to about 600 µg to the mammal.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the A_{2A} receptor is administered in a single dose.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the partial A_{2A} receptor agonist is administered by iv bolus.

The method of increasing patient tolerability during vasodilator induced myocardial stress perfusion imaging of a mammal may comprise administering caffeine and a radionuclide and the partial A_{2A} receptor agonist in an amount ranging from about 10 to about 600 µg wherein the partial A_{2A} receptor agonist is administered in less than about 10 seconds.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the partial A_{2A} receptor agonist is administered in an amount greater than about 10 µg.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the partial A_{2A} receptor agonist is administered in an amount greater than about 100 µg.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the partial A_{2A} receptor agonist is administered in an amount no greater than 600 µg.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the partial A_{2A} receptor agonist is administered in an amount no greater than 500 µg.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the partial A_{2A} receptor agonist is administered in an amount ranging from about 100 µg to about 500 µg.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the myocardium is examined for areas of insufficient blood flow following administration of the radionuclide and the partial A_{2A} receptor agonist.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the myocardium is examined for areas of insufficient blood flow following administration of the radionuclide and the partial A_{2A} receptor agonist wherein the myocardium examination begins within about 1 minute from the time the partial A_{2A} receptor agonist is administered.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the administration of the partial A_{2A} receptor agonist causes at least a 2.5 fold increase in coronary blood flow.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the administration of the partial A_{2A} receptor agonist causes at least a 2.5 fold increase in coronary blood flow that is achieved within about 1 minute from the administration of the partial A_{2A} receptor agonist.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the radionuclide and the partial A_{2A} receptor agonist are administered separately.

The method of increasing patient tolerability during vasodilator induced myocardial stress perfusion imaging of a mammal may comprise administering caffeine and a radionuclide and the partial A_{2A} receptor agonist in an amount ranging from about 10 to about 600 µg wherein the radionuclide and the partial A_{2A} receptor agonist are administered simultaneously.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the administration of the partial A_{2A} receptor agonist causes at least a 2.5 fold increase in coronary blood flow for less than about 5 minutes.

The method of increasing patient tolerability during vasodilator induced myocardial stress perfusion imaging of a mammal, may comprise administering caffeine and a radionuclide and the partial A_{2A} receptor agonist in an amount ranging from about 10 to about 600 µg wherein the administration of the partial A_{2A} receptor agonist causes at least a 2.5 fold increase in coronary blood flow for less than about 3 minutes.

The method of increasing patient tolerability during vasodilator induced myocardial stress perfusion imaging of a mammal, may comprise administering caffeine and Regadenoson in an amount ranging from about 10 to about 600 µg in a single iv bolus.

The method of increasing patient tolerability during vasodilator induced myocardial stress perfusion imaging of a mammal may comprise administering caffeine and Regadenoson in an amount ranging from about 100 to about 500 µg in a single iv bolus.

In all of the methods above, the mammal is typically a human.

In all of the methods above, the dose is typically administered in a single iv bolus.

In all of the method above, at least one radionuclide is administered before, with or after the administration of the A_{2A} receptor agonist to facilitate myocardial imaging.

The following are further aspects of this invention:
Caffeine for use in a method of increasing patient tolerability during vasodilator induced myocardial stress perfusion imaging of a human, comprising administering from about 50 mg to about 1000 mg caffeine, a radionuclide and a partial A_{2A} receptor agonist in an amount ranging from about 10 µg to about 600 µg wherein the myocardium is to be examined for areas of insufficient blood flow following administration of the radionuclide and the partial A_{2A} receptor agonist, wherein the partial A_{2A} receptor agonist is selected from the group consisting of CVT-3033, Regadenoson, and combinations thereof.
Caffeine for use in a method of increasing patient tolerability during vasodilator induced myocardial stress perfusion imaging of a human, comprising administering from about 50 mg to about 1000 mg, preferably 100 mg to about 500 mg caffeine and from about 10 µg to about 600 µg, preferably about 100 µg to about 500 µg Regadenoson to the human in a single iv bolus.

### DESCRIPTION OF THE FIGURES

Figure 1 depicts line graphs showing time course of coronary blood flow (CBF) following administration (twice) of regadenoson (5 µg/kg, i.v) (The dashed line indicates 2-fold increase in CBF). Values are Mean ± SEM.

Figure 2 plots the time course of coronary blood flow (CBF), in the absence and presence of caffeine, following administration of Regadenoson (5 µg/kg, i.v.). Panels A, B, C, and D represent the CBF in the absence or presence of caffeine at 1, 2, 4 and 10 mg/kg. Values are Mean ± SEM, #P < 0.05, compared with control.

Figure 3 shows the plasma regadenoson (top panel) and caffeine (bottom panel) concentrations following IV administration. Values are Mean ± SEM.

Figure 4 presents line graphs showing percentage changes in the maximum increase in CBF and in the duration of 2-fold Increase in CBF caused by regadenoson (5 µg/kg, IV). In the presence of caffeine, the maximum increases in CBF caused by regadenoson were not significantly altered, however, the durations of 2-fold increase in CBF caused by regadenoson were reduced in a dose-dependent manner. Values are Mean ± SEM, # P< 0.05, compared with control.

Figure 5 presents the result of the Tolerability Questionnaire discussed in Example 2.

### DESCRIPTION OF THE INVENTION

Potent partial A_{2A} agonists are useful as adjuncts in cardiac imaging when added either prior to dosing with an imaging agent or simultaneously with an imaging agent. Suitable imaging agents are ²⁰¹Thallium or ^{99m}Technetium-Sestamibi, ^{99mTc}teboroxime, and ^{99mtc}(III).

The compositions may be administered orally, intravenously, through the epidermis or by any other means known in the art for administering therapeutic agents with bolus IV administration being preferred.

New and potent partial A_{2A} agonists that increase CBF but do not significantly increase peripheral blood flow have been identified. The partial A_{2A} agonists, and especially Regadenoson and CVT-3033 have a rapid onset and a short duration when administered. An unexpected and newly identified benefit of these new compounds is that they are very useful when administered in a very small quantity in a single bolus intravenous injection. The partial A_{2A} receptor agonists can be administered in amounts as little as 10 µg and as high as 600 µg or more and still be effective few if any side-effects. An optimal intravenous dose will include from about 100 to about 500 µg of at least one partial A_{2A} receptor agonist. This amount is unexpectedly small when compared with adenosine which is typically administered in continuously by IV at a rate of about 140 µg/kg/min. Unlike adenosine, the same dosage of partial A_{2A} receptor agonists, an in particular, Regadenoson and CVT-3033 can be administered to a human patient regardless of the patient's weight. Thus, the administration of a single uniform amount of a partial A_{2A} receptor agonists by iv bolus for myocardial imaging is dramatically simpler and less error prone than the time and weight dependent administration of adenosine.

It has been surprisingly discovered that caffeine improves patient tolerability to partial A_{2A} receptor agonists administered during myocardial imaging. In particular, patient tolerability is improved when caffeine is administered to a patient either prior to or with the administration of the partial A_{2A} receptor agonist. Patient tolerability improvement is demonstrated by, for example, a reduction in CBF and/or by reports from human patients that demonstrate that caffeine administration improved their tolerance to the partial A_{2A} receptor agonist Regadenoson.

Caffeine can be administered to a mammal and preferably a human patient prior to administration of a partial A_{2A} receptor agonist. Prior administration refers to administration at a time before administration of the partial A_{2A} receptor agonist that allows a therapeutically effective amount of caffeine to remain in the mammal's blood at the time of the administration of the partial A_{2A} receptor agonist. More preferably, prior administration refers to administration of caffeine no greater than about 120 minutes before and even more preferably no greater than 30 minutes before administration of the partial A_{2A} receptor agonist.

Alternatively, caffeine can be administered at the same time as the partial A_{2A} receptor agonist. Towards this end, the caffeine can be incorporated into the partial A_{2A} receptor agonist containing pharmaceutical composition or it can be administered as a separate pharmaceutical composition.

Caffeine will be administered to mammals according to this invention in a therapeutically effective amount. The therapeutically effective amount will be an amount of caffeine that is sufficient to produce an improvement in a mammal's tolerance to the administration of an partial A_{2A} receptor agonist. Generally, a therapeutically effective amount will be a dose of caffeine ranging from about 50mg to about 1000mg. More preferably, the dose of caffeine will range from about 100mg to about 500mg. Most preferably, the dose of caffeine will range from about 200mg to about 400mg.

The caffeine may be administered to the mammal in a liquid or sold pharmaceutical dosage. As discussed above, the caffeine may be administered with or independently from the partial A_{2A} receptor agonist. If caffeine is administered with the partial A_{2A} receptor agonist, then it is preferred that the combination is administered as a single iv bolus. If caffeine is administered independently. i.e., separately from the partial A_{2A} receptor agonist, then the caffeine can be administered in any known manner including by way of a solid oral dosage form - tablet - by way of an iv infusion or iv bolus, or by way of a liquid such as a caffeine spiked liquid or by way of a naturally occurring caffeine containing liquid such as coffee or tea.

Pharmaceutical compositions may be formulated as solutions or lyophilized powders for parenteral administration. Powders may be reconstituted by addition of a suitable diluent or other pharmaceutically acceptable carrier prior to use. If used in liquid form the compositions of this invention are preferably incorporated into a buffered, isotonic, aqueous solution. Examples of suitable diluents are normal isotonic saline solution, standard 5% dextrose in water and buffered sodium or ammonium acetate solution. Such liquid formulations are suitable for parenteral administration, but may also be used for oral administration. It may be desirable to add excipients such as polyvinylpyrrolidinone, gelatin, hydroxy cellulose, acacia, polyethylene glycol, mannitol, sodium chloride, sodium citrate or any other excipient known to one of skill in the art to pharmaceutical compositions of this invention.

A first class of compounds that are potent and selective agonists for the A_{2A} adenosine receptor are 2-adenosine N-pyrazole compounds having the formula: 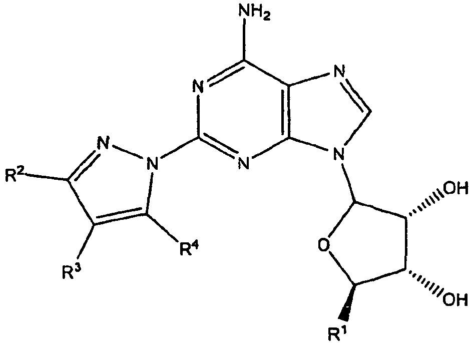 wherein

R¹ = CH₂OH, -CONR⁵R⁶;

R² and R⁴ are selected from the group consisting of H, C₁₋₆ alkyl and aryl, wherein the alkyl and aryl substituents are optionally substituted with halo, CN, CF₃, OR²⁰ and N(R²⁰)₂ with the proviso that when R² is not hydrogen then R⁴ is hydrogen, and when R⁴ is not hydrogen then R² is hydrogen;

R³ is independently selected from the group consisting of C₁₋₁₅ alkyl, halo, NO₂, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂,-CONR⁷R⁸, C₂₋₁₅ alkenyl, C₂₋₁₅ alkynyl, heterocyclyl, aryl, and heteroaryl, wherein the alkyl, alkenyl, alkynyl, aryl, heterocyclyl and heteroaryl substituents are optionally substituted with from I to 3 substituents independently selected from the group consisting of halo, alkyl, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR¹⁰SO₂R²², SO₂NR²⁰CO₂R²², OCONR²⁰SO₂R²², OC(O)R²⁰, C(O)OCH₂OC(O)R²⁰, and OCON(R²⁰)₂ and wherein the optional substituted heteroaryl, aryl, and heterocyclyl substituents are optionally substituted with halo, NO₂, alkyl, CF₃, amino, mono- or dialkylamino, alkyl or aryl or heteroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN, or OR²⁰;

R⁵ and R⁶ are each individually selected from H, and C₁-C₁₅ alkyl that is optionally substituted with from 1 to 2 substituents independently selected from the group of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ wherein each optional substituted heteroaryl, aryl, and heterocyclyl substituent is optionally substituted with halo, NO₂, alkyl, CF₃, amino, monoalkylamino, dialkylamino, alkylamide, arylamide, heteromylamide, NCOR¹², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN, and OR²⁰;

R⁷ and R⁸ are each independently selected from the group consisting of hydrogen, C₁₋₁₅ alkyl, C₂₋₁₅ alkenyl, C₂₋₁₅ alkynyl, heterocyclyl, aryl and heteroaryl, wherein the alkyl, alkenyl, alkynyl, aryl, heterocyclyl and heteroaryl substituents are optionally substituted with from 1 to 3 substituents independently selected from the group of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰ and OCON(R²⁰)₂ and wherein each optional substituted heteroaryl, aryl and heterocyclyl substituent is optionally substituted with halo, NO₂, alkyl, CF₃, amino, mono- or dialkylamino, alkyl or aryl or heteroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰₎₂, CN, and OR²⁰;

R²⁰ is selected from the group consisting of H, C₁₋₁₅ alkyl, C₂₋₁₅ alkenyl, C₂₋₁₅ alkynyl, heterocyclyl, aryl, and heteroaryl, wherein the alkyl, alkenyl, alkynyl, heterocyclyl, aryl, and heteroaryl substituents are optionally substituted with from 1 to 3 substituents independently selected from halo, alkyl, mono- or dialkylamino, alkyl or aryl or heteroaryl amide, CN, O-C₁₋₆ alkyl, CF₃, aryl, and heteroaryl; and

R²² is selected from the group consisting of C₁₋₁₅ alkyl, C₂₋₁₅ alkenyl, C₂₋₁₅ alkynyl, heterocyclyl, aryl, and heteroaryl, wherein the alkyl, alkenyl, alkynyl, heterocyclyl, aryl, and heteroaryl substituents are optionally substituted with from 1 to 3 substituents independently selected from halo, alkyl, mono- or dialkylamino, alkyl or aryl or heteroaryl amide, CN, O-C₁₋₆ alkyl, CF₃, aryl, and heteroaryl.

In a related group of compounds,
R³ is selected from the group consisting of C₁₋₁₅ alkyl, halo,CF₃, CN, OR²⁰, SR²⁰, S(O)R²², SO₂R²², SO₂N(R²⁰)₂, COR²⁰, CO₂R²⁰, -CONR⁷R⁸, aryl and heteroaryl wherein the alkyl, aryl and heteroaryl substituents are optionally substituted with from 1 to 3 substituents independently selected from the group consisting of halo, aryl, heteroaryl, CF₃, CN, OR²⁰, SR²⁰, S(O)R²², SO₂R²², SO₂N(R²⁰)₂, COR²⁰, CO₂R²⁰ or CON(R²⁰)₂, and each optional heteroaryl and aryl substituent is optionally substituted with halo, alkyl, CF₃ CN, and OR²⁰;
R⁵ and R⁶ are independently selected from the group of H and C₁-C₁₅ alkyl including one optional aryl substituent and each optional aryl substituent that is optionally substituted with halo or CF₃;
R⁷ is selected from the group consisting of C₁₋₁₅ alkyl, C₂₋₁₅ alkynyl, aryl, and heteroaryl, wherein the alkyl, alkynyl, aryl, and heteroaryl substituents are optionally substituted with from 1 to 3 substituents independently selected from the group consisting of halo, aryl, heteroaryl, CF₃, CN, OR²⁰, and each optional heteroaryl and aryl substituent is optionally substituted with halo, alkyl, CF₃ CN, or OR²⁰;
R⁸ is selected from the group consisting of hydrogen and C₁₋₁₅ alkyl;
R²⁰ is selected from the group consisting of H, C₁₋₄ alkyl and aryl, wherein alkyl and aryl substituents are optionally substituted with one alkyl substituent; and
R²² is selected from the group consisting of C₁₋₄ alkyl and aryl which are each optionally substituted with from 1 to 3 alkyl group.

In yet another related class of compounds,
R¹ is CH₂OH;
R³ is selected from the group consisting of CO₂R²⁰, -CONR⁷R⁸ and aryl where the aryl substituent is optionally substituted with from 1 to 2 substituents independently selected from the group consisting of halo, C₁₋₆ alkyl, CF₃ and OR²⁰;
R⁷ is selected from the group consisting of hydrogen, C₁₋₈ alkyl and aryl, where the alkyl and aryl substituents are optionally substituted with one substituent selected from the group consisting of halo, aryl, CF₃, CN, OR²⁰ and wherein each optional aryl substituent is optionally substituted with halo, alkyl, CF₃ CN, and OR²⁰;
R⁸ is selected from the group consisting of hydrogen and C₁₋₈ alkyl; and
R²⁰ is selected from hydrogen and C₁₋₄ alkyl.

In a still another related class of compounds,
R¹ = CH₂OH;
R³ is selected from the group consisting of CO₂R²⁰, -CONR⁷R⁸, and aryl that is optionally substituted with one substituent selected from the group consisting of halo, C ₁₋₃ alkyl and OR²⁰;
R⁷ is selected from of hydrogen, and C₁₋₃ alkyl;
R⁸ is hydrogen; and
R²⁰ is selected from hydrogen and C₁₋₄ alkyl.

In this preferred embodiment, R³ is most preferably selected from -CO₂Et and -CONHEt.

In yet another related class of compounds,
R¹ = -CONHEt,
R³ is selected from the group consisting of CO₂R²⁰, -CONR⁷R⁸, and aryl in that aryl is optionally substituted with from 1 to 2 substituents independently selected from the group consisting of halo, C₁₋₃ alkyl, CF₃ or OR²⁰;
R⁷ is selected from the group consisting of hydrogen, and C₁₋₈ alkyl that is optionally substituted with one substituent selected from the group consisting of halo, CF₃, CN or OR²⁰;
R⁸ is selected from the group consisting of hydrogen and C₁₋₃ alkyl; and R²⁰ is selected from the group consisting of hydrogen and C₁₋₄ alkyl.

In this more preferred embodiment, R⁸ is preferably hydrogen, R⁷ is preferably selected from the group consisting of hydrogen, and C₁₋₃, and R²⁰ is preferably selected from the group consisting of hydrogen and C₁₋₄ alkyl.

Specific useful compounds are selected from

ethyl 1-{9-[(4S,2R,3R,5R)-3,4-dihydroxy-5-(hydroxymethyl)oxolan-2-yl]-6-aminopurin-2-yl}pyrazole-4-carboxylate,

(4S,2R,3R,5R)-2-{6-amino-2-[4-(4-chlorophenyl)pyrazolyl]purin-9-yl}-5-(hydroxymethyl)oxolane-3,4-diol,

(4S,2R,3R,5R)-2-{6-amino-2-[4-(4-methoxyphenyl)pyrazolyl]purin-9-yl}-5-(hydroxymethyl)oxolane-3,4-diol,

(4S,2R,3R,5R)-2-{6-amino-2-[4-(4-methylphenyl)pyrazolyl]purin-9-yl}-5-(hydroxymethyl) oxolane-3,4-diol,

(1-{9-[(4S,2R,3R,5R)-3,4-dihydroxy-5-(hydroxymethyl)oxolan-2-yl]-6-aminopurin-2-yl}pyrazol-4-yl)-N-methylcarboxamide,

1-{9-[(4S,2R,3R,SR)-3,4-dihydroxy-5-(hydroxymethyl)oxolan-2-yl]-6-aminopurin-2-yl}pyrazole-4-carboxylic acid,

(1-{9-[(4S,2R,3R,5R)-3,4-dihydroxy-5-(hydroxymethyl)oxolan-2-yl]-6-aminopurin-2-yl}pyrazol-4-yl)-N,N-dimethylcarboxamide,

(1-{9-[(4S,2R,3R,5R)-3,4-dihydroxy-5-(hydroxymethyl)oxolan-2-yl]-6-aminopurin-2-yl}pyrazol-4-yl)-N-ethylcarboxamide,

1-{9-[(4S,2R,3R,SR)-3,4-dihydroxy-5-(hydroxymethyl)oxolan-2-yl]-6-aminopurin-2-yl}pyrazole-4-carboxamide,

1-{9-[(4S,2R,3R,5R)-3,4-dihydroxy-5-(hydroxymethyl)oxolan-2-yl]-6-aminopurin-2-yl}pyrazol-4-yl)-N-(cyclopentylmethyl)carboxamide,

(1-{9-[(4S,2R, 3R,5R)-3,4-dihydroxy-5-(hydroxymethyl)oxolan-2-yl]-6-aminopurin-2-yl pyrazol-4-yl)-N-[(4-chlorophenyl)methyl]carboxamide,

ethyl 2-[(1-{9-[(4S,2R,3F,5R)-3,4-dihydroxy-5-(hydroxymethyl)oxolan-2-yl]-6-aminopurin-2-yl}pyrazol-4-yl)carbonylamino]acetate, and mixtures thereof.

A second class of compounds that are potent and selective agonists for the A_{2A} adenosine receptor are 2-adenosine C-pyrazole compounds having the following formula: 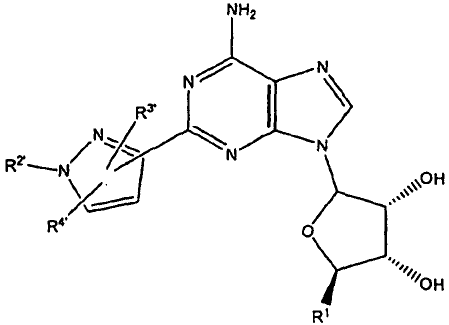 wherein

R¹ is as previously defined;

R^{2'} is selected from the group consisting of hydrogen, C₁₋₁₅ alkyl, C₂₋₁₅ alkenyl, C₂₋₁₅ alkynyl, heterocyclyl, aryl, and heteroaryl, wherein the alkyl, alkenyl, alkynyl, aryl, heterocyclyl, and heteroaryl substituents are optionally substituted with from 1 to 3 substituents independently selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ and wherein each optional heteroaryl, aryl, and heterocyclyl substituent is optionally substituted with halo, NO₂, alkyl, CF₃, amino, mono- or di- alkylamino, alkyl or aryl or heteroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN, or OR²⁰;

R^{3'}, R^{4'} are individually selected from the group consisting of hydrogen, C₁₋₁₅ alkyl, C₂₋₁₅ alkenyl, C₂₋₁₅ alkynyl, heterocyclyl, aryl, and heteroaryl, halo, NO₂, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ wherein the alkyl, alkenyl, alkynyl, aryl, heterocyclyl, and heteroaryl substituents are optionally substituted with from I to 3 substituents individually selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, NR²⁰C(NR²⁰)NHR²³, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ and wherein each optional heteroaryl, aryl, and heterocyclyl substituent is optionally substituted with halo, NO₂, alkyl, CF₃, amino, mono- or di- alkylamino, alkyl or aryl or heteroaryl amide, NCOR²², NR²⁰SO₂R²², COR²⁰, CO₂R²⁰, CON(R²⁰)₂*,* NR²⁰CON(R²⁰)₂, OC(O)R²⁰, OC(O)N(R²⁰)₂, SR²⁰, S(O)R²², SO₂R²², SO₂N(R²⁰)₂, CN, or OR²⁰; and

R⁵ R⁶, R²⁰, and R²² are also as previously defined,

with the proviso that when R¹ = CH₂OH, R^{3'} is H, R^{4'} is H, the pyrazole ring is attached through C^{4'}, and R^{2'} is not H.

When the compound has one of the following formulas: 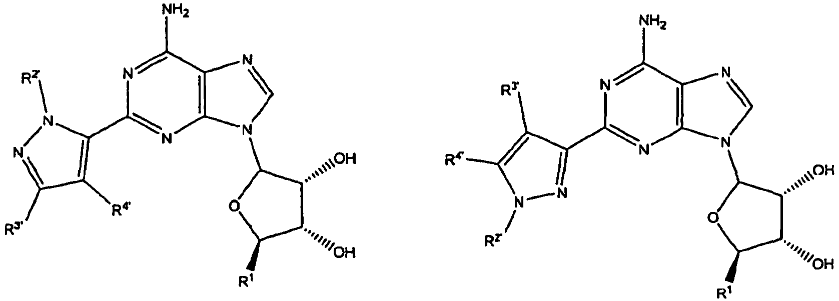 then it is preferred that R¹ is -CH₂OH; R^{2'} is selected from the group consisting of hydrogen, C₁₋₈ alkyl wherein the alkyl is optionally substituted with one substituent independently selected from the group consisting of aryl, CF₃, CN, and wherein each optional aryl substituent is optionally substituted with halo, alkyl, CF₃ or CN; and R^{3'} and R^{4'} are each independently selected from the group consisting of hydrogen, methyl and more preferably, R^{3'} and R^{4'} are each hydrogen.

When the compound has the following formula: 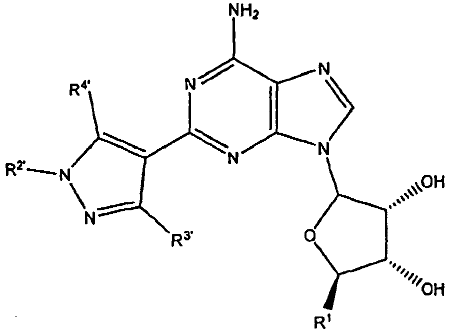 then it is preferred that R¹ is -CH₂OH; R^{2'} is selected from the group consisting of hydrogen, and C₁₋₆ alkyl optionally substituted by phenyl. More preferably, R^{2'} is selected from benzyl and pentyl; R^{3'} is selected from the group consisting of hydrogen, C₁₋₆ alkyl, aryl, wherein the alkyl, and aryl substituents are optionally substituted with from 1 to 2 substituents independently selected from the group consisting of halo, aryl, CF₃, CN, and wherein each optional aryl substituent is optionally substituted with halo, alkyl, CF₃ or CN; and R^{4'} is selected from the group consisting of hydrogen and C₁₋₆ alkyl, and more preferably, R^{4'} is selected from hydrogen and methyl.

A more specific class of compounds is selected from the group consisting of

(4S,2R,3R,5R)-2-{6-amino-2-[1-benzylpyrazol-4-yl]purin-9-yl}-5-(hydroxymethyl)oxolane-3,4-diol,

(4S,2R,3R,SR)-2-[6-amino-2-(1-pentylpyrazol-4-yl)purin-9yl]-5-(hydroxymethyl)oxolane-3,4-diol,

(4S,2R,3R,5R)-2-[6-amino-2-(1-methylpyrazol-4-yl)purin-9-yl]-5-(hydroxymethyl)oxolane-3,4-diol,

(4S,2R,3R,SR)-2-{6-amino-2-[1-(methylethyl)pyrazol-4-yl]purin-9-yl}-5-(hydroxymethyl)oxolane-3,4-diol,

(4S,2R,3R,5R)-2-{6-amino-2-[1-(3-phenylpropyl)pyrazol-4-yl]purin-9-yl}-5-(hydroxymethyl)oxolane-3,4-diol,

(45,2R,3R,5R)-2-{6-amino-2-[1-(4-t-butylbenzyl)pyrazol-4-yl]purin-9-yl}-5-(hydroxymethyl)oxolane-3,4-diol,

(4S,2R,3R,5R)-2-(6-amino-2-pyrazol-4-yipurin-9-yl)-5-(hydroxymethyl)oxolane-3,4-diol,

(4S,2R,3R,5R)-2-{6-amino-2-[1-pent-4-enylpyrazol-4-yl]purin-9-yl}-5-(hydroxymethyl)oxolane-3,4-diol,

(4S,2P,3R,5R)-2-{6-amino-2-[1-decylpyrazol-4-yl]purin-9-yl}-5-(hydroxymethyl)oxolane-3,4-diol,

(4S,2R,3R,5R)-2-{6-amino-2-[1-(cyclohexylmethyl)pyrazol-4-yl]purin-9-yl}-5-(hydroxymethyl)oxolane-3,4-diol,

(4S,2R,3R,SR)-2-{6-amino-2-[1-(2-phenylethyl]pyrazol-4-yl]purin-9-yl}-5-(hydroxymethyl)oxolane-3,4-diol,

(4S,2R,3R,5R)-2-{6-amino-2-[1-(3-cyclohexylpropyl)pyrazol-4-yl]purin-9-yl}-5-(hydroxymethyl)oxolane-3,4-diol,

(4S,2R,3R,5R)-2-{6-amino-2-[1-(2-cyclohexylethyl)pyrazol-4-yl]purin-9-yl}-5-(hydroxymethyl)oxolane-3,4-diol, and combinations thereof.

A very useful and potent and selective agonists for the A_{2A} adenosine receptor is Regadenoson or (1-{9-[(4S,2R,3R,5R)-3,4-dihydroxy-5-(hydroxymethyl)oxolan-2-yl]-6-aminopurin-2-yl}pyrazol-4-yl)-N-methylcarboxamide which has the formula:

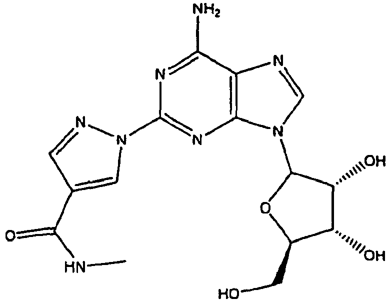

Another preferred compound that is useful as a selective partial A_{2A}-adenosine receptor agonist with a short duration of action is a compound of the formula: 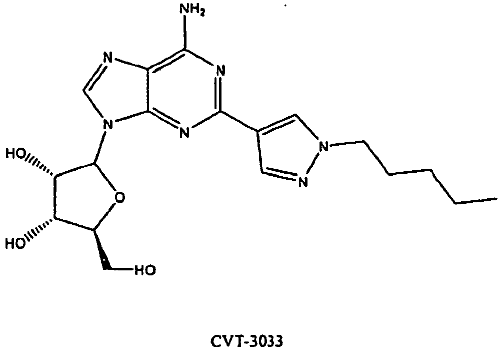 CVT-3033 is particularly useful as an adjuvant in cardiological imaging.

The first and second classes of compounds identified above are described in more detail in U.S. Patent Nos. 6,403,567 and 6,214,807.

The following definitions apply to terms as used herein.

"Halo" or "Halogen" - alone or in combination means all halogens, that is, chloro (Cl), fluoro (F), bromo (Br), iodo (I).

"Hydroxyl" refers to the group -OH.

"Thiol" or "mercapto" refers to the group -SH.

"Alkyl" - alone or in combination means an alkane-derived radical containing from 1 to 20, preferably 1 to 15, carbon atoms (unless specifically defined). It is a straight chain alkyl, branched alkyl or cycloalkyl. Preferably, straight or branched alkyl groups containing from 1-15, more preferably 1 to 8, even more preferably 1-6, yet more preferably 1-4 and most preferably 1-2, carbon atoms, such as methyl, ethyl, propyl, isopropyl, butyl, t-butyl and the like. The term "lower alkyl" is used herein to describe the straight chain alkyl groups described immediately above. Preferably, cycloalkyl groups are monocyclic, bicyclic or tricyclic ring systems of 3-8, more preferably 3-6, ring members per ring, such as cyclopropyl, cyclopentyl, cyclohexyl, adamantyl and the like. Alkyl also includes a straight chain or branched alkyl group that contains or is interrupted by a cycloalkyl portion. The straight chain or branched alkyl group is attached at any available point to produce a stable compound. Examples of this include, but are not limited to, 4-(isopropyl)-cyclohexylethyl or 2-methyl-cyclopropylpentyl. A substituted alkyl is a straight chain alkyl, branched alkyl, or cycloalkyl group defined previously, independently substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like.

"Alkenyl" - alone or in combination means a straight, branched, or cyclic hydrocarbon containing 2-20, preferably 2-17, more preferably 2-10, even more preferably 2-8, most preferably 2-4, carbon atoms and at least one, preferably 1-3, more preferably 1-2, most preferably one, carbon to carbon double bond. In the case of a cycloalkyl group, conjugation of more than one carbon to carbon double bond is not such as to confer aromaticity to the ring. Carbon to carbon double bonds may be either contained within a cycloalkyl portion, with the exception of cyclopropyl, or within a straight chain or branched portion. Examples of alkenyl groups include ethenyl, propenyl, isopropenyl, butenyl, cyclohexenyl, cyclohexenylalkyl and the like. A substituted alkenyl is the straight chain alkenyl, branched alkenyl or cycloalkenyl group defined previously, independently substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, carboxy, alkoxycarbonyl, aryloxycarbonyl, heteroaryloxycarbonyl, or the like attached at any available point to produce a stable compound.

"Alkynyl" - alone or in combination means a straight or branched hydrocarbon containing 2-20, preferably 2-17, more preferably 2-10, even more preferably 2-8, most preferably 2-4, carbon atoms containing at least one, preferably one, carbon to carbon triple bond. Examples of alkynyl groups include ethynyl, propynyl, butynyl and the like. A substituted alkynyl refers to the straight chain alkynyl or branched alkenyl defined previously, independently substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like attached at any available point to produce a stable compound.

"Alkyl alkenyl" refers to a group -R-CR'=CR"' R"", where R is lower alkyl, or substituted lower alkyl, R', R"', R"" may independently be hydrogen, halogen, lower alkyl, substituted lower alkyl, acyl, aryl, substituted aryl, hetaryl, or substituted hetaryl as defined below.

"Alkyl alkynyl" refers to a group -RC≡CR' where R is lower alkyl or substituted lower alkyl, R' is hydrogen, lower alkyl, substituted lower alkyl, acyl, aryl, substituted aryl, hetaryl, or substituted hetaryl as defined below.

"Alkoxy" denotes the group -OR, where R is lower alkyl, substituted lower alkyl, acyl, aryl, substituted aryl, aralkyl, substituted aralkyl, heteroalkyl, heteroarylalkyl, cycloalkyl, substituted cycloalkyl, cycloheteroalkyl, or substituted cycloheteroalkyl as defined.

"Alkylthio" denotes the groups-SR, -S(O)ₙ₌₁₋₂-R, where R is lower alkyl, substituted lower alkyl, aryl, substituted aryl, aralkyl or substituted aralkyl as defined herein.

"Acyl" denotes groups -C(O)R, where R is hydrogen, lower alkyl substituted lower alkyl, aryl, substituted aryl and the like as defined herein.

"Aryloxy" denotes groups -OAr, where Ar is an aryl, substituted aryl, heteroaryl, or substituted heteroaryl group as defined herein.

"Amino" denotes the group NRR', where R and R' may independently by hydrogen, lower alkyl, substituted lower alkyl, aryl, substituted aryl, hetaryl, or substituted hetaryl as defined herein or acyl.

"Amido" denotes the group -C(O)NRR', where R and R' may independently by hydrogen, lower alkyl, substituted lower alkyl, aryl, substituted aryl, hetaryl, substituted hetaryl as defined herein.

"Carboxyl" denotes the group -C(O)OR, where R is hydrogen, lower alkyl, substituted lower alkyl, aryl, substituted aryl, hetaryl, and substituted hetaryl as defined herein.

"Aryl" - alone or in combination means phenyl or naphthyl optionally carbocyclic fused with a cycloalkyl of preferably 5-7, more preferably 5-6, ring members and/or optionally substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like.

"Substituted aryl" refers to aryl optionally substituted with one or more functional groups, *e.g*., halogen, lower alkyl, lower alkoxy, alkylthio, acetylene, amino, amido, carboxyl, hydroxyl, aryl, aryloxy, heterocycle, hetaryl, substituted hetaryl, nitro, cyano, thiol, sulfamido and the like.

"Heterocycle" refers to a saturated, unsaturated, or aromatic carbocyclic group having a single ring (*e.g*., morpholino, pyridyl or furyl) or multiple condensed rings (*e.g*., naphthpyridyl, quinoxalyl, quinolinyl, indolizinyl or benzo[b]thienyl) and having at least one hetero atom, such as N, O or S, within the ring, which can optionally be unsubstituted or substituted with, *e.g*., halogen, lower alkyl, lower alkoxy, alkylthio, acetylene, amino, amido, carboxyl, hydroxyl, aryl, aryloxy, heterocycle, hetaryl, substituted hetaryl, nitro, cyano, thiol, sulfamido and the like.

"Heteroaryl" - alone or in combination means a monocyclic aromatic ring structure containing 5 or 6 ring atoms, or a bicyclic aromatic group having 8 to 10 atoms, containing one or more, preferably 1-4, more preferably 1-3, even more preferably 1-2, heteroatoms independently selected from the group O, S, and N, and optionally substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like. Heteroaryl is also intended to include oxidized S or N, such as sulfinyl, sulfonyl and N-oxide of a tertiary ring nitrogen. A carbon or nitrogen atom is the point of attachment of the heteroaryl ring structure such that a stable aromatic ring is retained. Examples of heteroaryl groups are pyridinyl, pyridazinyl, pyrazinyl, quinazolinyl, purinyl, indolyl, quinolinyl, pyrimidinyl, pyrrolyl, oxazolyl, thiazolyl, thienyl, isoxazolyl, oxathiadiazolyl, isothiazolyl, tetrazolyl, imidazolyl, triazinyl, furanyl, benzofuryl, indolyl and the like. A substituted heteroaryl contains a substituent attached at an available carbon or nitrogen to produce a stable compound.

"Heterocyclyl" - alone or in combination means a non-aromatic cycloalkyl group having from 5 to 10 atoms in which from 1 to 3 carbon atoms in the ring are replaced by heteroatoms of O, S or N, and are optionally benzo fused or fused heteroaryl of 5-6 ring members and/or are optionally substituted as in the case of cycloalkyl. Heterocycyl is also intended to include oxidized S or N, such as sulfinyl, sulfonyl and N-oxide of a tertiary ring nitrogen. The point of attachment is at a carbon or nitrogen atom. Examples of heterocyclyl groups are tetrahydrofuranyl, dihydropyridinyl, piperidinyl, pyrrolidinyl, piperazinyl, dihydrobenzofuryl, dihydroindolyl, and the like. A substituted hetercyclyl contains a substituent nitrogen attached at an available carbon or nitrogen to produce a stable compound.

"Substituted heteroaryl" refers to a heterocycle optionally mono or poly substituted with one or more functional groups, *e.g*., halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Aralkyl" refers to the group -R-Ar where Ar is an aryl group and R is lower alkyl or substituted lower alkyl group. Aryl groups can optionally be unsubstituted or substituted with, *e.g*., halogen, lower alkyl, alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Heteroalkyl" refers to the group -R-Het where Het is a heterocycle group and R is a lower alkyl group. Heteroalkyl groups can optionally be unsubstituted or substituted with *e.g*., halogen, lower alkyl, lower alkoxy, alkylthio, acetylene, amino, amido, carboxyl, aryl, aryloxy, heterocycle, substituted heterocycle, hetaryl, substituted hetaryl, nitro, cyano, thiol, sulfamido and the like.

"Heteroarylalkyl" refers to the group -R-HetAr where HetAr is an heteroaryl group and R lower alkyl or substituted lower alkyl. Heteroarylalkyl groups can optionally be unsubstituted or substituted with, *e.g*., halogen, lower alkyl, substituted lower alkyl, alkoxy, alkylthio, acetylene, aryl, aryloxy, heterocycle, substituted heterocycle, hetaryl, substituted hetaryl, nitro, cyano, thiol, sulfamido and the like.

"Cycloalkyl" refers to a divalent cyclic or polycyclic alkyl group containing 3 to 15 carbon atoms.

"Substituted cycloalkyl" refers to a cycloalkyl group comprising one or more substituents with, *e.g*., halogen, lower alkyl, substituted lower alkyl, alkoxy, alkylthio, acetylene, aryl, aryloxy, heterocycle, substituted heterocycle, hetaryl, substituted hetaryl, nitro, cyano, thiol, sulfamido and the like.

"Cycloheteroalkyl" refers to a cycloalkyl group wherein one or more of the ring carbon atoms is replaced with a heteroatom (*e.g*., N, O S or P).

Substituted cycloheteroalkyl" refers to a cycloheteroalkyl group as herein defined which contains one or more substituents, such as halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Alkyl cycloalkyl" denotes the group -R-cycloalkyl where cycloalkyl is a cycloalkyl group and R is a lower alkyl or substituted lower alkyl. Cycloalkyl groups can optionally be unsubstituted or substituted with *e.g*. halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Alkyl cycloheteroalkyl" denotes the group -R-cycloheteroalkyl where R is a lower alkyl or substituted lower alkyl. Cycloheteroalkyl groups can optionally be unsubstituted or substituted with *e.g.* halogen, lower alkyl, lower alkoxy, alkylthio, amino, amido, carboxyl, acetylene, hydroxyl, aryl, aryloxy, heterocycle, substituted heterocycle, hetaryl, substituted hetaryl, nitro, cyano, thiol, sulfamido and the like.

The first class of compounds identified above can be prepared as outlined in Schemes 1-4.

Compounds having the general formula **IV** can be prepared as shown in Scheme 1.

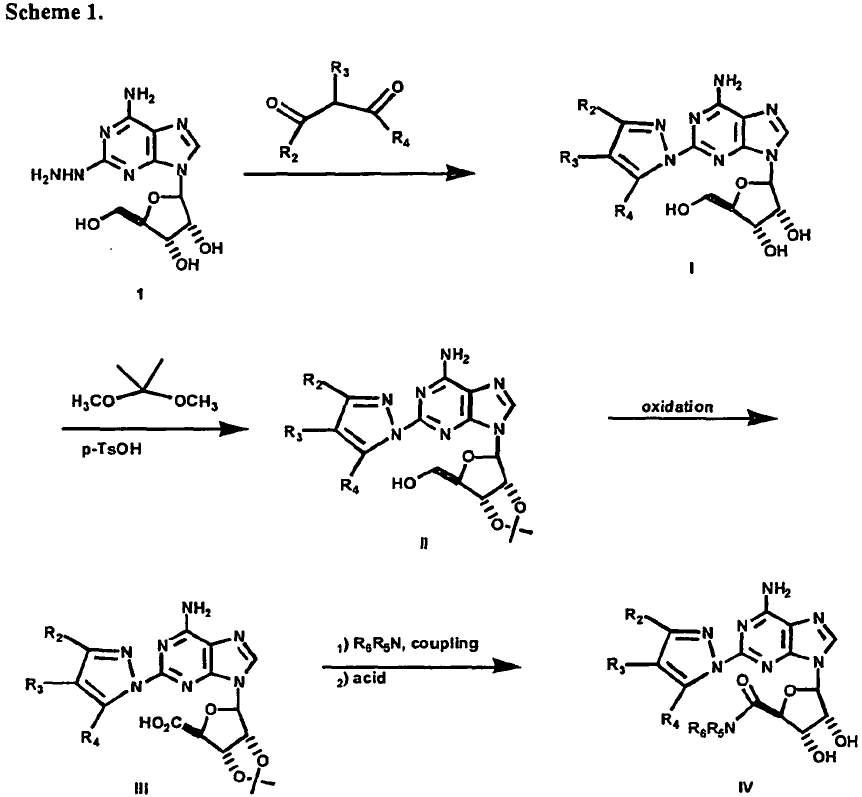

Compound I can be prepared by reacting compound 1 with appropriately substituted 1,3-dicarbonyl in a mixture of AcOH and MeOH at 80°C (Holzer et al., J Heterocycl. Chem. (1993) 30, 865). Compound II, which can be obtained by reacting compound I with 2,2-dimethoxypropane in the presence of an acid, can be oxidized to the carboxylic acid III, based on structurally similar compounds using potassium permanganate or pyridinium chlorochromate (M. Hudlicky, (1990) Oxidations in Organic Chemistry, ACS Monographs, American Chemical Society, Washington D. C.). Reaction of a primary or secondary amine having the formula HNR⁶R⁷, and compound III using DCC (M. Fujino et al., Chem. Pharm. Bull. (1974), 22,1857), PyBOP (J. Martinez et al., J. Med. Chem. (1988) 28, 1874) or PyBrop (J. Caste et al. Tetrahedron, (1991), 32, 1967) coupling conditions can afford compound IV.

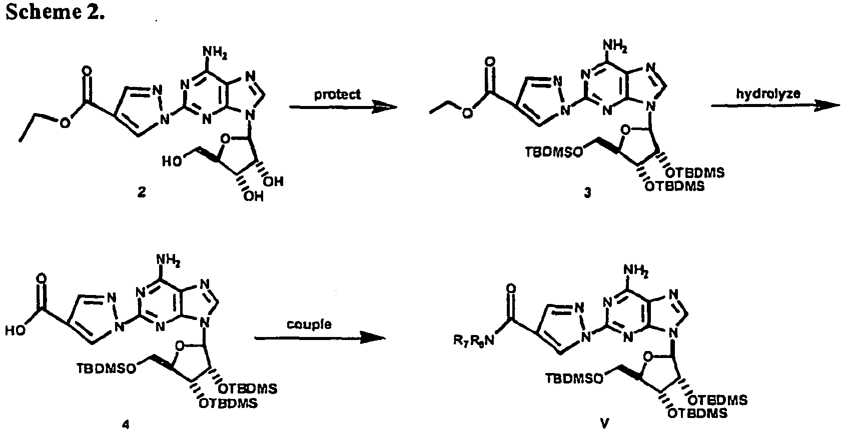

Compound V can be prepared as shown in Scheme 2. The Tri TBDMS derivative 4 can be obtained by treating compound **2** with TBDMSCl and imidazole in DMF followed by hydrolysis of the ethyl ester using NaOH. Reaction of a primary or secondary amine with the formula HNR⁶R⁷, and compound **4** using DCC (M. Fujino et al., Chem. Pharm. Bull. (1974), 22, 1857), PyBOP (J. Martinez et al., J. Med. Chem. (1988) 28, 1874) or PyBrop (J. Caste et al. Tetrahedron, (1991), 32, 1967) coupling conditions can afford compound V.

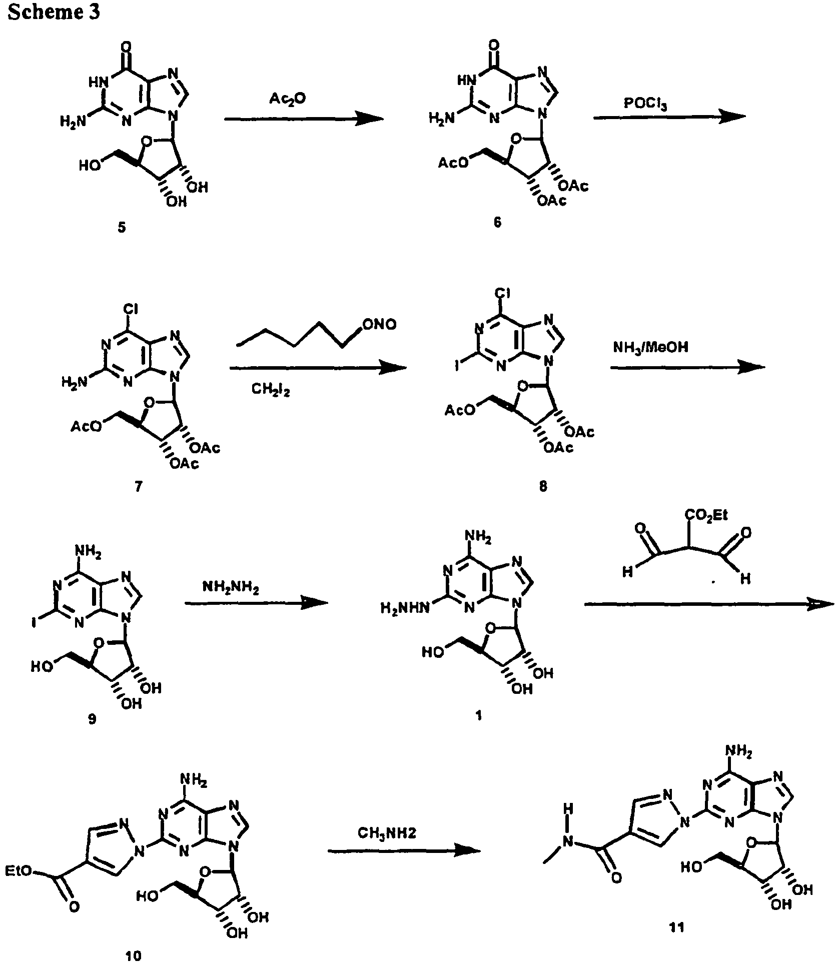

A specific synthesis of compound **11** is illustrated in Scheme 3. Commercially available guanosine **5** was converted to the triacetate **6** as previously described (M. J. Robins and B. Uznanski, Can. J. Chem. (1981), 59, 2601-2607). Compound **7**, prepared by following the literature procedure of Cerster et al. (J. F. Cerster, A. F. Lewis, and R.K. Robins, Org. Synthesis, 242-243), was converted to compound **9** in two steps as previously described (V. Nair et al., J. Org. Chem., (1988), 53, 3051-3057). Compound **1** was obtained by reacting hydrazine hydrate with compound **9** in ethanol at 80°C. Condensation of compound **1** with ethoxycarbonylmalondialdehyde in a mixture of AcOH and MeOH at 80°C produced compound **10.** Heating compound **10** in excess methylamine afforded compound 11.

The synthesis of 1,3-dialdehyde VII is described in Scheme 4.

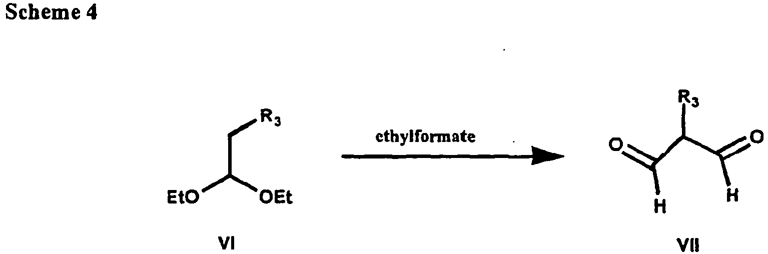

Reaction of 3,3-diethoxypropionate or 3,3-diethoxypropionitrile or 1,1-diethoxy-2-nitroethane VI (R₃ = CO₂R, CN or NO₂) with ethyl or methyl formate in the presence of NaH can afford the dialdehyde VII (Y. Yamamoto et al., J. Org. Chem. (1989) 54, 4734).

The second class of compound described above may be prepared by as outlined in Schemes 5-9. As shown in Scheme 5, compounds having the general formula VIII: 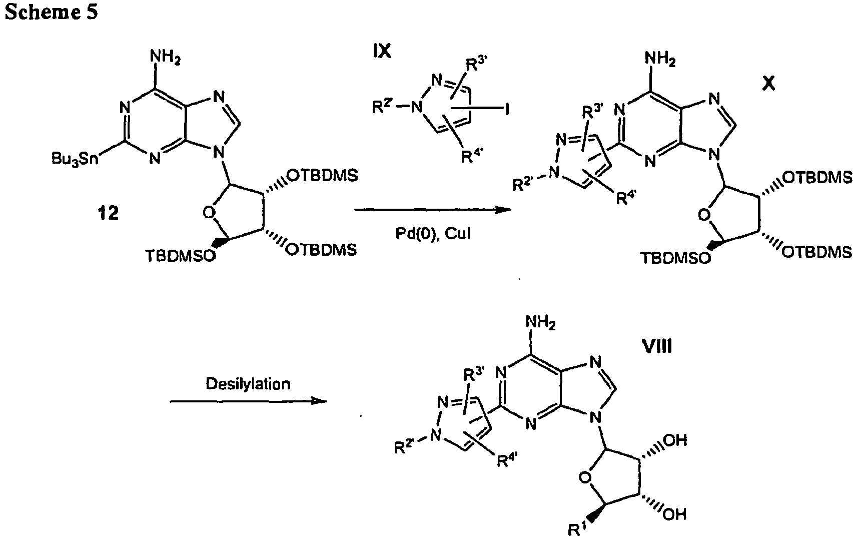 were prepared by the palladium mediated coupling of compound **12** with halo-pyrazoles represented by the formula IX (synthesis shown in Scheme 8) in the presence or absence of copper salts (K. Kato et. al. J. Org. Chem. 1997, 62, 6833-6841; Palladium Reagents and Catalysts-Innovations in Organic Synthesis, Tsuji, John Wiley and Sons, 1995) followed by de-protection with either TBAF or NH₄F (Markiewicz et. al Tetrahedron Lett.(1988), 29, 1561). The preparation of compound **12** has been previously described (K. Kato et. al. J. Org. Chem. 1997, 62, 6833-6841) and is outlined in Scheme 11.

Compounds with general formula XIV can be prepared as shown in Scheme 6.

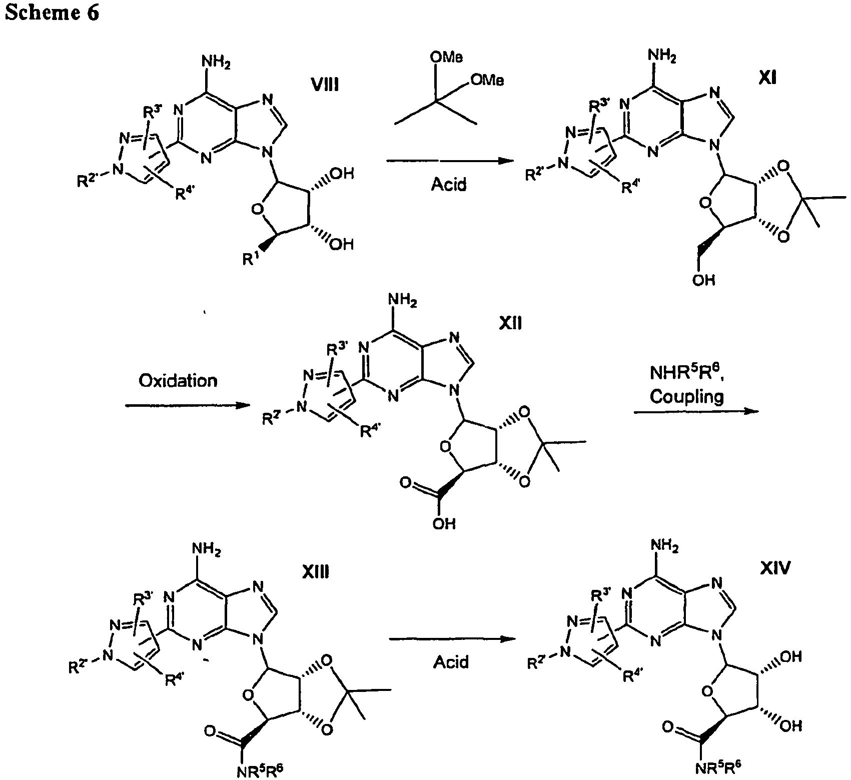

Compound XI, which can be obtained by reacting VII with 2,2-dimethoxypropane in presence of an acid, can be oxidized to the carboxylic acid XII, based on structurally similar compounds, using potassium permanganate or pyridinium chlorochromate etc. (Jones et.al., J Am. Chem. Soc.(1949), 71, 3994.; Hudlicky, Oxidations in organic chemistry, American Chemical Society, Washington D. C., 1990).

Reaction of a primary or secondary amine of the formula NHR⁵R⁶, and compound XII using DCC (Fujino et.al., Chem. Pharm. Bull. (1974), 22, 1857), PyBOP (J. Martinez et. al., J Med. Chem. (1988), 28, 1967) or PyBrop (J. Caste et.al. Tetrahedron, (1991), 32, 1967) coupling conditions can afford compound XIII.

Deprotection of compound XIII can be performed by heating with 80% aq. acetic acid (T. W. Green and P. G. M. Wuts, (1991), Protective Groups in Organic Synthesis, A, Wiley-Interscience publication) or with anhydrous HCl (4N) to obtain compound of the general formula XIII.

Alternatively, compounds with the general formula VIII can also be prepared by Suzuki type coupling as shown in Scheme 7.

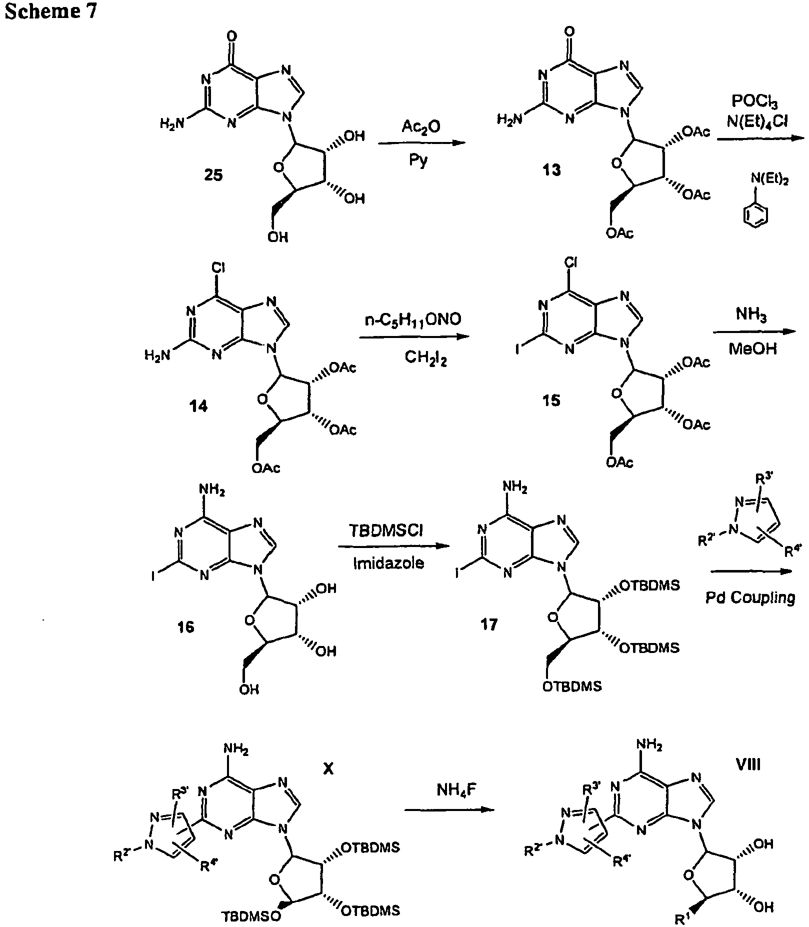

2-Iodoadenosine 16 can be prepared in four steps from guanosine 25 following literature procedures (M. J. Robins et al. Can. J. Chem. (1981), 59, 2601-2607; J. F. Cerster et.al. Org. Synthesis, ---242-243; V. Nair at. al., J. Org. Chem., (1988), 53, 3051-3057). Palladium mediated Suzuki coupling of **16** with appropriately substituted pyrazole-boronic acids in presence of a base can provide final compounds with general formula VIII (A.Suzuki, Acc.Chem.Res) (1982), 15, 178). If necessary, 2', 3', 5' hydroxyls on 6 can be protected as TBDMS ethers prior to Suzuki coupling.

Compounds with the general formula IX can be either commercially available or prepared following the steps shown in Scheme 8.

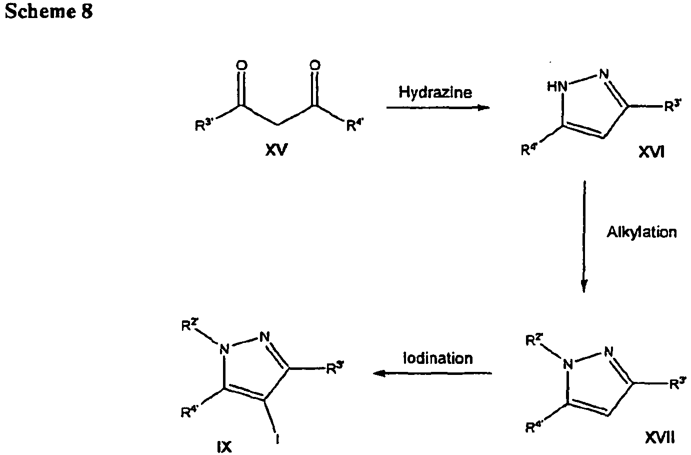

Condensation of 1,3-diketo compounds of the formula XV with hydrazine in an appropriate solvent can give pyrazoles with the general formula XVI (R.H.Wiley et al., Org.Synthsis, Coll.Vol IV (1963), 351*.* These pyrazoles can be N-alkylated with various alkyl halides to give compounds of the formula XVII which on iodination give 4-iodo derivatives with the general formula IX (R. Huttel et.al. Justus Liebigs Ann.Chem.(1955), 593, 200).

5-iodopyrazoles with the general formula XXI can be prepared following the steps outlined in Scheme 9.

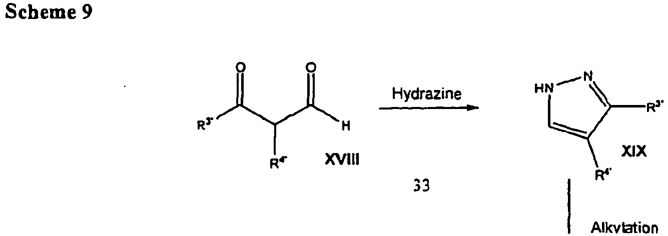

Condensation of 1,3-diketo compounds of the formula XVIII with hydrazine in an appropriate solvent can give pyrazoles with the general formula XIX. These pyrazoles can be N-allcylated with various alkyl halides to give compounds of the formula XX. Abstraction of 5-H with a strong base followed by quenching with iodine can provide 5-iodo derivatives with general formula XXI (F. Effenberger et. al. J. Org. Chem. (1984), 49, 468'n.

4- or 5- iodopyrazoles can be transformed into corresponding boronic acids as shown in the Scheme 10.

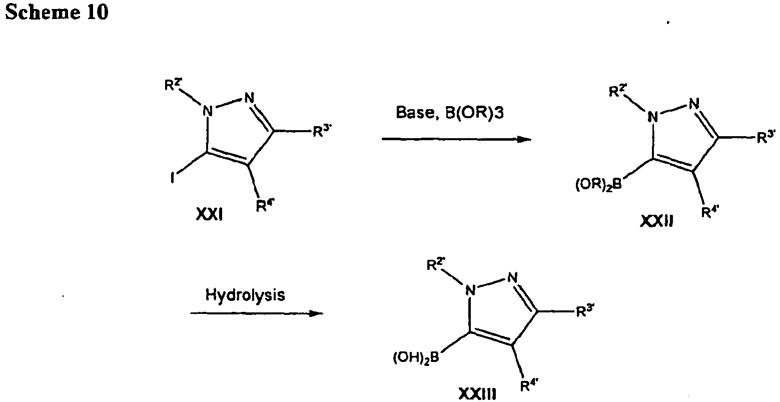

Transmetallation with n-buLi followed by treatment with trimethylborate can give compounds with the general formula XXII which on hydrolysis can provide boronic acids with the general formula XXIII (F. C. Fischer et.al. RECUEIL (1965), 84, 439).

As shown in Scheme 11 below, 2-Stannyladenosine **12** was prepared in three steps from the commercially available 6-chloropurine riboside following literature procedure (K. Kato et.al., J. Org. Chem. (1997), 62, 6833-6841).

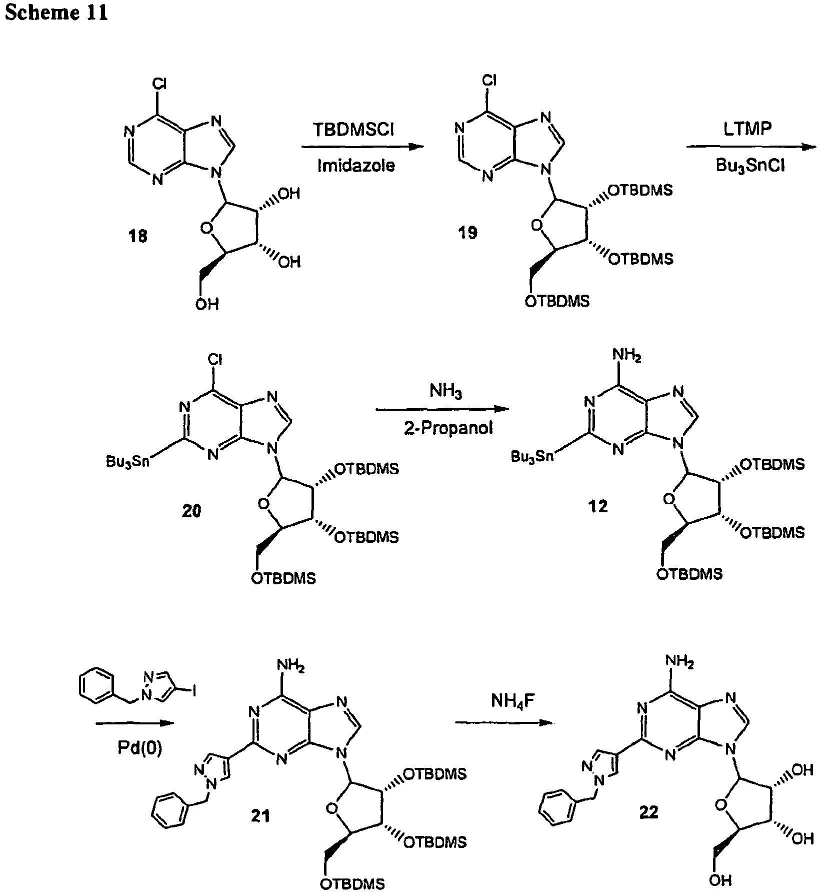

Tri TBDMS derivative was obtained by treating **18** with TBDMSCI and imidazole in DMF. Lithiation with LTMP followed by quenching with tri n-butyltin chloride gave exclusively 2-stannyl derivative **20.** Ammonolysis in 2-propanol gave 2-stannyladenosine 12. Stille coupling of 12 with 1-benzyl-4-iodopyrazole in presence of Pd(PPh₃)₄ and CuI resulted in **21** (K. Kato et.al., J. Org. Chem. (1997), 62, 6833-6841). Deprotection of silyl groups on 2',3' and 5' hydroxyls with 0.5 M ammonium fluoride in methanol gave 22 in good yield.

The methods used to prepare the compounds described herein are not limited to those described above. Additional methods can be found in the following sources (J. March, Advanced Organic Chemistry; Reaction Mechanisms and Studies (1992), A Wiley Interscience Publications; and J. Tsuji, Palladium reagents and catalysts-Innovations in organic synthesis, John Wiley and Sons, 1995).

If the final compound contains a basic group, an acid addition salt may be prepared. Acid addition salts of the compounds are prepared in a standard manner in a suitable solvent from the parent compound and an excess of acid, such as hydrochloric, hydrobromic, sulfuric, phosphoric, acetic, maleic, succinic, or methane sulfonic. The hydrochloric salt form is especially useful. If the final compound contains an acidic group, cationic salts may be prepared. Typically the parent compound is treated with an excess of an alkaline reagent, such as hydroxide, carbonate or alkoxide, containing the appropriate cation. Cations such as Na⁺, K⁺, Ca⁺² and NH₄⁺ are examples of cations present in pharmaceutically acceptable salts. Certain of the compounds form inner salts or zwittcrions which may also be acceptable.

### EXAMPLE 1

Effects of caffeine (1 to 10 mg/kg) on coronary vasodilation and changes in hemodynamics by Regadenoson (5 µg/kg, IV) were determined in conscious dogs. Caffeine dose-dependently attenuated the duration of coronary vasodilation, but not the peak increase in coronary hyperemia induced by Regadenoson. Caffeine (4 and 10 mg/kg) significantly reduced the effects of Regadenoson on mean arterial pressure and heart rate. The results suggest that caffeine consumption immediately prior to pharmacologic stress testing with an A_{2A} adenosine receptor agonist may abbreviate the duration of coronary vasodilation caused by the drug.
Abbreviation list:
CBF: coronary blood flow
MAP: mean arterial pressure
HR: heart rate
LVSP: left ventricular systolic pressure

### METHODS

Sixteen chronically instrumented male mongrel dogs weighing from 22-30 kg were used in the study. The animal protocol was approved by the Institutional Animal Care and Use Committee of New York Medical College and conforms to the Guide for the Care and Use of Laboratory Animal by the United States National Institutes of Health.

### Surgical Procedures

Dogs were sedated with acepromazine (0.3 mg/kg, IM) and anesthetized with pentobarbital sodium (25 mg/kg, IV). After intubation, dogs were artificially ventilated with room air. A thoracotomy was made in the fifth intercostal space using sterile techniques. A Tygon catheter (Cardiovascular Instruments, Wakefield, MA) was inserted into the descending thoracic aorta and another one was inserted into the left atrium. In 9 dogs, an ultrasound flow transducer (Transonic Systems, Ithaca, NY) was placed around the left circumflex coronary artery. A solid-state pressure gauge (P6.5, Konisberg Instruments, Pasadena, CA) was placed into the left ventricle through the apex. The chest was closed in layers. The catheters and wires were tunneled subcutaneously and externalized through the skin at the back of the dog's neck. Dogs were allowed to recover from the surgery before experiments were performed, and were trained to lie on a table.

### Coronary Blood Flow and Hemodynamic Measurements

Phasic arterial pressure was measured by connecting the aortic catheter to a strain gauge transducer (P23 ID, LDS Test and Measurement, Valley View, OH). Left ventricular pressures were measured by the solid pressure gauge. CBF (mL/min) was measured from an ultrasound flow transducer using a Transonic flowmeter (T206, Transonic Systems, Ithaca, NY). Two indices were used to describe the Regadenoson-induced coronary vasodilation: 1) the maximum increase in CBF and 2) the duration of the 2-fold increase in CBF (the period of time that CBF was elevated to a level ≥ 2-fold of baseline CBF). All pressure and flow data were acquired and analyzed using a Ponemah System (Version 3.30 or 4.20, LDS Test and Measurement, Valley View, OH). MAP and HR were calculated from phasic blood pressure, and LV dP/dt_{Max} was calculated from the left ventricular systolic pressure.

### Experimental Protocols

On the day of an experiment, a dog was placed on a table, where it lay quietly during the experiment. A catheter was inserted into a peripheral vein in the leg and attached to an infusion line to administer drugs without disturbing the dog. The experiment was begun after MAP, HR and CBF were stable.

### Effects of Caffeine Alone on MAP and HR, and Determination of Plasma Caffeine Concentrations (Part I):

Three experiments were performed on each dog in the group. In each experiment, a dog received an IV injection (over 1 to 3 min) of caffeine at a dose of 2, 4 or 10 mg/kg. Each dog received up to 3 doses of caffeine (on different days) in a random manner. MAP and HR were recorded continuously for 120 min and 3 mL of blood was taken from the aortic catheter at 2.5, 5, 15, 30, 60, 90 and 120 min following administration of caffeines, for measurements of plasma caffeine concentrations.

### Effects of Caffeine on Regadenoson-Induced Coronary Vasodilation and Changes in Hemodynamics (Part II):

Each dog received an IV injection of 5 µg/kg of Regadenoson. Forty-five min later, 1 mg/kg of caffeine (IV) was administered. About 45 min after the injection of caffeine, a second-injection of Regadenoson was given. LVSP, LV dP/df_{Max}, MAP, HR and CBF were recorded continuously. Blood samples were taken from the left atrial catheter at 1, 3, 5, 15, 30, 45 and 60 min following injections of Regadenoson.

On subsequent days, the protocol and blood sampling were repeated in the same dogs with different doses of caffeine (2, 4 or 10 mg/kg).

In 4 dogs, two doses of Regadenoson (5 µg/kg, IV) were given 90 min apart (without blood sampling) to determine if there is tachyphylaxis of Regadenoson-induced coronary vasodilation.

### Drugs

Regadenoson was supplied by CV Therapeutics, Inc. as a sterile stock solution (Lot#: 803604, 0.08 mg/mL), that was made using 15% Propylene Glycol (pH 7) and was diluted in normal saline before injection. Caffeine was purchased from Sigma-Aldrich (St. Louis, MO), and was dissolved in normal saline (10 mg/mL).

### Statistical Analysis

The statistical significance of a difference between the value of a parameter at baseline and at the indicated time point after drug administration was determined using a One-Way Repeated Measures ANOVA followed by Tukey's Test. The statistical significance of a difference between responses to Regadenoson in the absence and presence of caffeine was determined using a Two-Way Repeated Measures ANOVA followed by Tukey's Test. Results with p < 0.05 were considered to be significant. A computer-based software package (SigmaStat 2.03) was used for statistical analysis. All data are presented as Mean ± SEM.

### RESULTS

### Effects of Caffeine Alone on MAP and HR, and Plasma Caffeine Concentrations

An IV injection of caffeine at 2 mg/kg caused no significant changes in MAP and HR. Caffeine at 4 mg/kg caused a significant increase in MAP by ~12 mm Hg at both 2.5 and 5 min after injection, without a significant change in HR. Caffeine at 10 mg/kg caused an insignificant increase in MAP (5 to 9 mm Hg at 2.5, 5 and 15 min, p>0.05), but did decrease HR by 16 to 24 beats/min from 30 to 120 min after injection. Plasma caffeine concentrations remained within a relatively narrow range from 30 to 120 min following a caffeine injection (Table 1). Based on these results, it was concluded that 45 min after caffeine administration was optimal for determining the effects of caffeine on Regadenoson-induced changes in CBF and hemodynamics.

**Table 1 Effects of Caffeine (IV) on MAP and HR, and Caffeine Plasma Concentrations in Conscious Dogs**

| | Baseline | 2.5 min | 5 min | 15 min | 30 min | 60 min | 90 min | 120 min |
|---|---|---|---|---|---|---|---|---|
| MAP (mm Hg) | | | | | | | | |
| 2 mg/kg | 107±4 | 110±5 | 108±3 | 106±4 | 104±4 | 112±5 | 111±7 | 109±6 |
| 4 mg/kg | 97±3 | 109±6* | 108±6* | 99±4 | 103±4 | 104±2 | 108±4* | 104±4 |
| 10 mg/kg | 99±4 | 109±5 | 107±3 | 105±4 | 101±3 | 107±4 | 104±6 | 102±2 |

| HR (beats/min) | | | | | | | | |
|---|---|---|---|---|---|---|---|---|
| 2 mg/kg | 95±6 | 95±5 | 91±5 | 85±6 | 81±7 | 90±9 | 87±5 | 88±6 |
| 4 mg/kg | 100:1:8 | 104±5 | 102±4 | 88±6 | 90±7 | 85±7* | 90±7 | 86±5 |
| 10 mg/kg | 103±5 | 100±4 | 101±4 | 93±5 | 87±5* | 83±2* | 80±5* | 80±4* |

| Caffeine Levels (µM) | | | | | | | | |
|---|---|---|---|---|---|---|---|---|
| 2 mg/kg | - | 19±0.98 | 15±0.29 | 12±0.19 | 11±0.10 | 9.9±0.11 | 9.1±0.11 | 8.7±0.18 |
| 4 mg/kg | - | 35±0.93 | 28±1.28 | 22±0.89 | 20±0.74 | 17±1.07 | 17±0.64 | 16±0.98 |
| 10 mg/kg | - | 76±3.00 | 67±2.19 | 52±1.37 | 47±2.14 | 45±1.22 | 41±1.78 | 37±1.78 |

| | | | | | | | | |
|---|---|---|---|---|---|---|---|---|
| MAP: Mean arterial pressure. HR: Heart rate. Mean ± SEM, n = 5 (n = 6 for caffeine levels). Baselines are values before the injection of caffeine. * p < 0.05, compared with baseline. | | | | | | | | |

### Effects of Caffeine on Regadenoson-Induced Coronary Vasodilation

### Time Control Group:

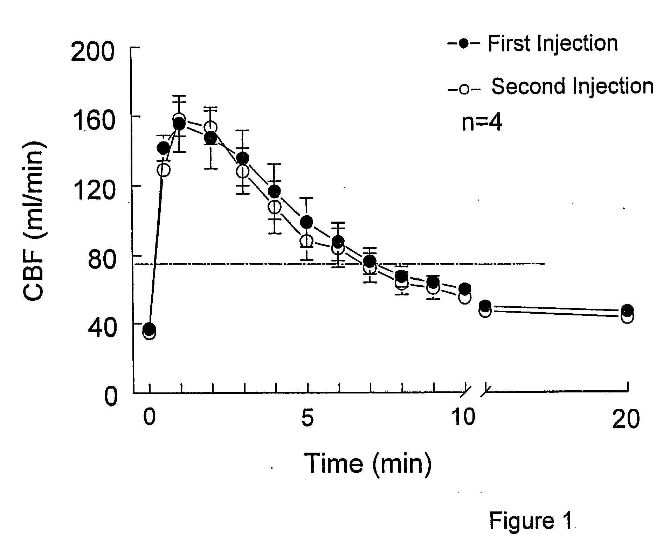

In 4 dogs, an IV injection of Regadenoson (5 µg/kg) caused a significant increase in CBF. The maximum CBF increased from a baseline value of 37 ± 1 to 178 ± 17 mL/min, and the duration of 2-fold increase in CBF was 401 ± 45 sec. A second-injection of Regadenoson resulted in an identical coronary vasodilation 90 min later (Figure 1). The maximum CBF increased from a baseline value of 35 ± 1 to 176 ± 6 mL/min, and the duration of 2-fold increase in CBF was 395 ± 43 sec. There were no statistically significant differences in baseline CBFs, in the maximum CBFs or in the duration of 2-fold increase in CBF caused by the two injections of Regadenoson (Figure 1).

### Effects of Caffeine on Regadenoson-Induced Coronary Vasodilation:

In the absence of caffeine, an IV injection of Regadenoson (5 µg/kg) increased CBF from a baseline value of 34 ± 2 to a peak of 191 ± 7 mL/min, and the duration of the 2-fold increase in CBF caused by Regadenoson was 515 ± 71 sec (n=8).

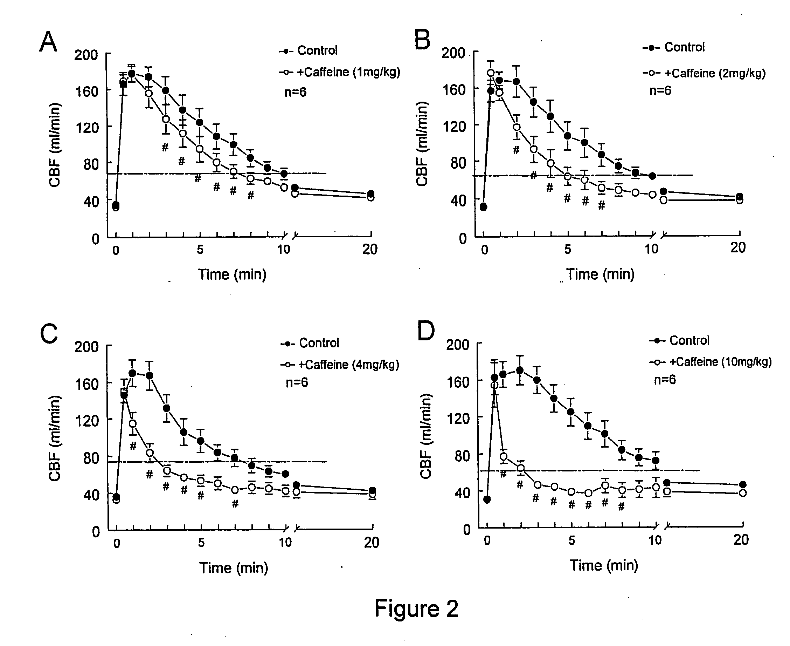

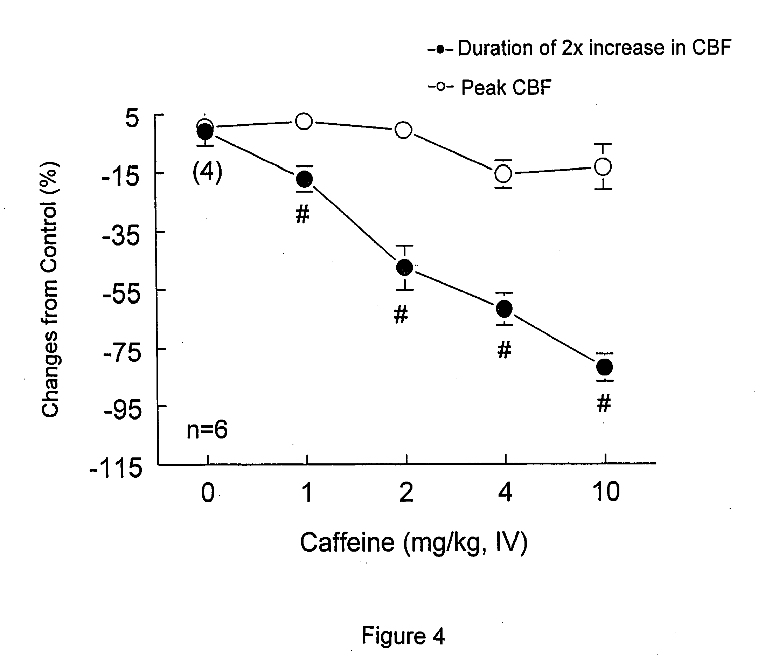

Baseline values of CBFs were not significantly different before and after caffeine treatment (45 min after 1, 2, 4, and 10 mg/kg administration) (Figure 2, Time 0). In the presence of caffeine at 1, 2, 4 and 10 mg/kg, the maximum increases in CBF caused by Regadenoson were not significantly reduced from control (in the absence of caffeine). The maximum increases in CBF induced by Regadenoson were changed by only 2 ± 3, -0.7 ± 3, -16 ± 5 and -13 ± 8%, respectively, in the presence of caffeine at 1, 2, 4 and 10 mg/kg (all p > 0.05, Figure 2). In contrast, the durations of the 2-fold increase in CBF caused by Regadenoson were significantly reduced at all dosages of caffeine tested. Reductions of the duration of 2-fold increase in CBF were 17 ± 4, 48 ± 8, 62 ± 5 and 82 ± 5% from control, respectively, in the presence of caffeine at 1, 2, 4 and 10 mg/kg (all p < 0.05) (Figures 4). However, the Regadenoson-increased CBF still remained at ≥ 2-fold baseline levels for ≥ 3 min in the presence of 1, 2 and 4 mg/kg caffeine (Figure 2).

### Plasma Concentrations of Regadenoson and Caffeine:

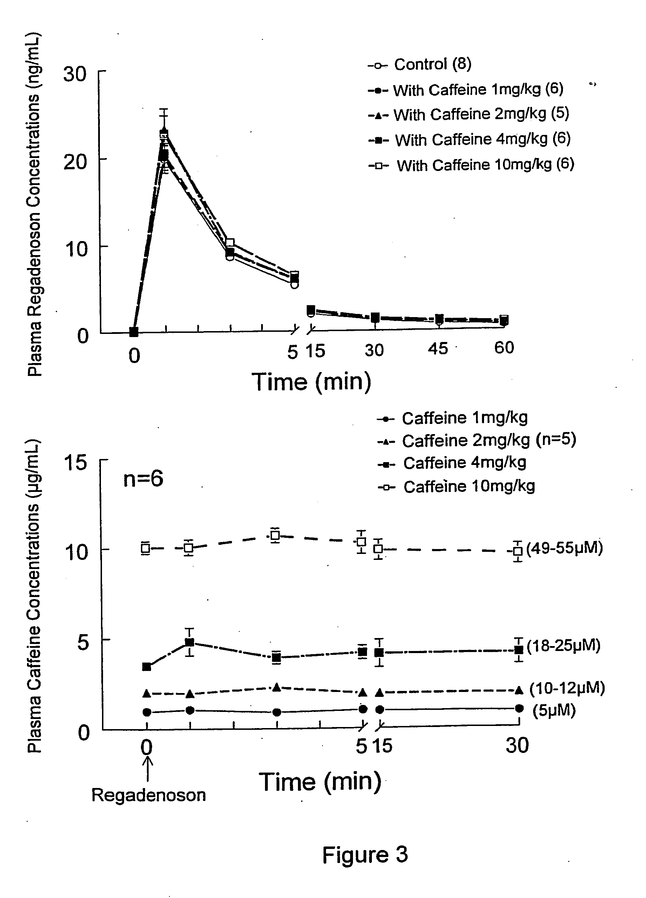

In the absence of caffeine, an IV injection of Regadenoson (5 µg/kg) caused a shortlasting increase in the plasma Regadenoson concentration, which reached at a peak at ∼1 min and decreased rapidly. Pharmacokinetic profiles of Regadenoson were not changed by caffeine at 1, 2, 4 or 10 mg/kg (Figure 3).

Plasma caffeine concentrations were 5 ± 0.2, 10 ± 0.6,18 ± 0.8 and 52 ±1.8 µM, respectively, at 45 min following administration of caffeine at 1, 2, 4 and 10 mg/kg and immediately before the second injection of Regadenoson (Time 0 in the bottom panel in Figure 21). Plasma caffeine concentrations remained at relatively steady levels from the time of preinjection (Time 0) to 30 min following the second injection of Regadenoson (Figure 3, the bottom panel).

### Effects of Caffeine on Regadenoson-Induced Changes in Hemodynamics

Table 2 shows the values of MAP and HR at different time points following administration of Regadenoson either in the absence or presence of caffeine at 1, 2, 4 and 10 mg/kg (The peak responses are not included). Caffeine at 1, 2, 4 or 10 mg/kg did not alter hemodynamics significantly at 45 min following caffeine administration as shown in Table 2 (the baseline values for control and caffeine at 1, 2, 4 and 10 mg/kg).

**Table 2 Effects of Caffeine on Regadenoson (5 µg/kg, IV)-Induced Changes in MAP and HR in Conscious Dogs**

| | Baseline | 0.5 min | 1 min | 2 min | 3 min | 4 min | 5 min | 10 min | 15 min | 20 min |
|---|---|---|---|---|---|---|---|---|---|---|
| MAP (mm Hg) | | | | | | | | | | |
| Control | 104±3 | 97±2 | 93±3* | 92±4* | 924:3* | 94±3* | 96±3* | 97±4 | 96±4 | 96±3 |
| Caffeine (1 mg(kg) | 109±5 | 105±3 | 100±4 | 102±4 | 101±5 | 105±4 | 104±3 | 104±4 | 106±4† | 102±4 |
| Control | 97±3 | 89±5 | 89±5 | 91±5 | 91±3 | 93±4 | 90±3 | 91±2 | 96±3 | 97±3 |
| Caffeine (2 mg/kg) | 110±6† | 106±7† | 102±7† | 104±7† | 106±5† | 105±7† | 103±6† | 106±5† | 107±7† | 111±8† |
| Control | 110±3 | 107±6 | 95±5* | 99±4* | 98±4* | 100±2 | 100±2 | 100±2 | 101±4 | 102±4 |
| Caffeine (4 mg/kg) | 112±3 | 109±5† | 107±5† | 107±4† | 109±3† | 112±3† | 111±5† | 109±3† | 107±3 | 103±1 |
| Control | 99±3 | 93±3 | 86±4* | 89±4* | 89±4* | 92±4 | 92±4 | 95±4 | 93±6 | 98±5 |
| Caffeine(10 mg/kg) | 106±3 | 116±7† | 115±4† | 112±5† | 111±4† | 112±6† | 111±4† | 110±4† | 113±5† | 111±5† |

| HR (bpm) | | | | | | | | | | |
|---|---|---|---|---|---|---|---|---|---|---|
| Control | 84±6 | 138±10* | 144±13* | 142±9* | 131±9* | 125±8* | 121±8* | 100±7 | 94±7 | 89±7 |
| Caffeine(1 mg/kg) | 74±5 | 126±7* | 135±9* | 131±12* | 119±9* | 110±4*† | 106±7*† | 89±7 | 87±7 | 81±8 |
| Control | 83±7 | 160±3* | 145±7* | 150±4* | 137±5* | 127±64* | 129±6* | 104±5 | 104±6 | 93±7 |
| Caffeine (2 mg/kg) | 75±5 | 121±10*† | 125±10*† | 122±5*† | 110±3*† | 106±4*† | 97±3† | 84±5† | 85±6† | 84±5 |
| Control | 89±7 | 166±18* | 163±8* | 158±6* | 141±4* | 131±6* | 128±7* | 113±5 | 102±6 | 101±6 |
| Caffeine (4 mg/kg) | 81±9 | 126±12*† | 114±11*† | 106±12*† | 102±7† | 94±8† | 94±7† | 85±8† | 85±8 | 87±7† |
| Control | 76±4 | 149±15* | 144±7* | 148±5* | 135±4* | 130±5* | 127±6* | 105±4 | 98±3 | 99±7 |
| Caffeine (10mg/kg) | 78±6 | 115±12*† | 102±6*† | 106±11*† | 96±7† | 94±8† | 93±5† | 88±7† | 88±6 | 86±4 |

| | | | | | | | | | | |
|---|---|---|---|---|---|---|---|---|---|---|
| MAP: Mean arterial pressure. HR: Heart rate. Mean ± SEM, n° 6 (Caffeine 1 mg/kg n = 7, Caffeine 2mg/kg: n = 5 for MAP). Baselines are values before the injection of regadenoson. The baselines for caffeine at 1, 2, 4 and 10mg/kg were the values at 45 min after injection of caffeine. * p 0.05, compared with baseline. p < 0.05, compared with control. Note: In the presence of 2 mg/kg caffeine, values of MAP at all time points were significantly higher than control, however, the delta changes in MAP following IV injection of regadenoson were not statistically different from those in control. | | | | | | | | | | |

An IV injection of Regadenoson (5 µg/kg) caused a mild decrease in MAP. Regadenoson decreased MAP (peak) by 15 ± 2% from a baseline value of 102 ± 2 mm Hg in the absence of caffeine (n=9). In the presence of caffeine at 1 and 2 mg/kg, the peak decrease in MAP caused by Regadenoson was unchanged (13 ± 2% vs. 13 ± 1% from baseline, respectively). However, in the presence of 4 mg/kg caffeine, Regadenoson decreased peak MAP by only 2 ± 5% from baseline. In the presence of 10 mg/kg caffeine, Regadenoson increased MAP, but insignificantly, by 9 ± 6% from baseline.

An IV injection of Regadenoson (5 µg/kg) caused an increase in HR lasting for 8 to 9 min. Regadenoson increased HR (peak) by 114 ± 14% from a baseline value of 80 ± 4 beats/min (n=9). Caffeine at 1 mg/kg did not markedly alter the Regadenoson-induced tachycardia. Peak HR increased by 124 ± 12% from baseline. Caffeine at 2, 4 or 10 mg/kg significantly attenuated the Regadenoson-induced tachycardia in a dose-dependent manner. Peak HRs increased by 109 ± 21%, 79 ± 20%, and 74 ± 16% from baseline, respectively (all p < 0.05, compared to control).

Regadenoson decreased LVSP (peak) by 9 ± 1% from a baseline value of 139 ± 5 mm Hg (n=8). In the presence of caffeine at 1 and 2 mg/kg, Regadenoson still significantly decreased LVSP by 9 ± 3% and 6 ± 2% from baseline, respectively. In the presence of 4 mg/kg of caffeine, Regadenoson caused no a significant decrease in LVSP (1 ± 5% decrease from control, p > 0.05), while in the presence of 10 mg/kg caffeine, Regadenoson significantly increased LVSP (11 ± 7% increase from control).

An IV injection of 5 µg/kg of Regadenoson caused an increase in LV dP/dt_{Max}. Regadenoson increased LV dP/dtₘₐₓ (peak) by 65 ± 7% from a baseline value of 3240 ± 196 mm Hg/s. The effects of caffeine on the Regadenoson-induced increase in LV dP/dt_{Max} were inconsistent. The increase in LV dP/dt_{Max} caused by Regadenoson was slightly greater in the presence of caffeine at 1 mg/kg. In the presence of caffeine at 2 and 4 mg/kg, the Regadenoson-induced increase in LV dP/dt_{Max} was slightly smaller. The Regadenoson-induced increase in LV dP/dt_{Max} was not altered in the presence of 10 mg/kg caffeine.

Both the magnitude of increase in CBF and the duration of coronary vasodilation are important for accurate diagnosis in myocardial perfusion imaging. The most important finding of the study is that caffeine attenuates the duration of coronary vasodilation, but not the peak increase in CBF in response to Regadenoson. Thus, the duration of an A_{2A} receptor-mediated coronary vasodilation is more sensitive than peak CBF to antagonism by caffeine.

Caffeine is a non-specific and unselective antagonist of all adenosine receptor subtypes. The affinities (Ki) of caffeine for human adenosine A₁, A_{2A}, A_{2B} and A₃ receptors are 12, 2.4, 13 and 80 µM, respectively (Fredholm et al. (1999). Pharmacol Rev, 51:83-133). A number of studies have shown that caffeine can attenuate coronary vasodilation induced by adenosine (Smits et al. (1990) Clin Pharmacol Ther, 48:410-8; Kubo et al. (2004) J Nucl Med,45:730-8*;* Lapeyre et al. (2004) J Nucl Cardiol, 11:506-11), by dipyridamole (Smits et al. (1991) J Nucl Med, 32:1538-41; Kubo et al. (2004) J Nucl Med,45:730-8; Lapeyre et al. (2004) J Nucl Cardiol, 11:506-11) and by an A_{2A} receptor agonist, ATL-146e (Riou et al. (2002) J Am Coll Cardiol, 40:1687-94) in humans and dogs. Thus, the action of caffeine can result in false-negative myocardial perfusion imaging in studies using these stress agents (Smits et al. (1991) J Nucl Med, 32:1538-41). However, one report indicated that caffeine did not alter adenosine-induced coronary hyperemia measured by fractional flow reserve in patients with coronary artery disease (Aqel et al. (2004) Am J Cardiol, 93:343-6).

The present results reveal for the first time that caffeine attenuates the Regadenoson-induced coronary hyperemia in a unique pattern: caffeine selectively attenuates the duration of Regadenoson-induced coronary vasodilation in a dose-dependent manner, but does not markedly alter the maximum increase in CBF. Caffeine at doses of 1 to 10 mg/kg did not reduce the peak plasma Regadenoson concentrations, or change the pharmacokinetic profile of Regadenoson. The differing affinities of A_{2A} receptor and pharmacokinetic profiles of Regadenoson and caffeine might explain the unique pattern of attenuation of coronary hyperemia caused by Regadenoson in the presence of caffeine. Immediately after injection, Regadenoson molecules could bind most of the A_{2A} receptors in the coronary circulation, thereby causing a similar maximum increase in CBF in the presence of all doses of caffeine. Shortly after injection, plasma Regadenoson concentrations decreased rapidly but plasma caffeine concentrations remained relatively constant. Therefore, as caffeine molecules occupy more A_{2A} receptors, the increase in CBF after the peak response to Regadenoson would decrease more rapidly in the presence of caffeine, thereby shortening the duration of coronary vasodilation caused by Regadenoson. Although these results show that caffeine caused a dose-dependent attenuation of the duration of Regadenoson-induced coronary vasodilation in conscious dogs, the Regadenoson-increased CBF remained at ≥ 2-fold of baseline levels for ≥ 3 min in the presence of caffeine at 1, 2 and 4 mg/kg (equivalent to consumption of 1 to 2 cups of coffee). More recently, it has been reported that one 8-oz cup of coffee taken 1 h prior to adenosine administration did not mask the presence or severity of a reversible defect studied by single-photon emission computed tomography (Zoghbi et al. (2006) J Am Coll Cordiol, 47:2296-302).

Desensitization of the A_{2A} receptor has been reported in cell-based experimental models (Anand-Srivastava et al. (1989) Mol Cell Endocrinol, 62:273-9, Ramkumar et al. (1991) Mol Pharmacol, 40:639-47). However, a related study demonstrated that three successive doses of 1.0 µg/kg Regadenoson (5 to 10 min apart) caused similar peak increases in CBF in conscious dogs (Trochu et al. (2003) J Cardiovasc Pharmacol, 41:132-9). Furthermore, in the present study, time control experiments were performed on four conscious dogs to determine if there is tachyphylaxis of the Regadenoson-induced coronary vasodilation. The results showed that there were no significant differences either in the maximum increases in CBF or in the duration of 2-fold increase in CBF induced by two injections of Regadenoson (Figure 1). Thus, the attenuated coronary hyperemia induced by Regadenoson in the presence of caffeine is most likely due to the competitive antagonism of A_{2A} receptors by caffeine.

The present study also showed that IV injection of Regadenoson caused mild decreases in MAP (Table 2) and LVSP, and modest increases in HR (Table 2) and LV dP/dt_{Max} in conscious dogs. The Regadenoson-induced changes in MAP and HR in the present study were consistent with related studies. (Trochu et al. (2003) J Cardiovasc Pharmacol, 41:132-9, Zhao et al. (2003) J Pharmacol Exp Ther, 307:182-9) which have indicated that the mild decrease in MAP induced by Regadenoson is due to dilation of peripheral vessels. This was evidenced by the reduction of total peripheral resistance (TPR) and dilation of vessels in the lower body by Regadenoson (Zhao et al. (2003) J Pharmacol Exp Ther, 307:182-9).

Caffeine has been shown to attenuate the dipyridamole-induced increase in blood pressure in humans in a dose-dependent manner (Smits et al. (1991) Clin Pharmacol Ther, 50:529-37). The present study further confirmed that caffeine caused a dose-dependently attenuation of hypotension induced by Regadenoson, a novel adenosine A_{2A} receptor agonist, in conscious dogs It was reported that adenosine could increase sympathetic nerve activity in humans, thereby causing tachycardia (Biaggioni et al. (1991) Circulation, 83:1668-75). The present results showed that an IV injection of Regadenoson caused a significant tachycardia in conscious dogs, and are consistent with related studies (Trochu et al. (2003) J Cardiovasc Pharmacol, 41:132-9, Zhao et al. (2003) J Pharmacol Exp Ther, 307:182-9). More importantly, one recent study indicated that the Regadenoson-induced tachycardia in awake rats is directly mediated by sympathoexcitation (Dhalla et al. (2006) J Pharmacol Exp Ther, 316:695-702), in which the Regadenoson-induced tachycardia was abolished by hexamethonium (a ganglionic blocker). The present study demonstrated that caffeine attenuated Regadenoson-induced tachycardia in a dose-dependent manner in conscious dogs. However, the mechanism(s) for the reduction by caffeine of tachycardia induced by Regadenoson remains to be determined.

In summary the result of the example above indicate that doses of 1 to 10 mg/kg IV caffeine
(1) did not alter baseline CBF and hemodynamics at 45 min, when caffeine plasma concentrations were as high as 52 ± 2 µM;
(2) did not significantly reduce the Regadenoson-induced peak increases in CBF;
(3) caused a dose-dependent decrease in the duration of the Regadenoson-induced coronary vasodilation; and
(4) blunted the Regadenoson-induced sinus tachycardia and hypotension.

### EXAMPLE 2

### Objectives:

The primary objective was to evaluate the effect of a 200-mg oral dose of caffeine on the regadenoson-induced increase in myocardial blood flow (MBF), measured approximately 2 hours after caffeine ingestion. Secondary objectives included the following:
- To evaluate the regadenoson-induced heart rate (HR) response with and without prior caffeine
- To evaluate the relationship between the regadenoson-induced increase in MBF and HR changes, and whether it is altered by oral caffeine
- To evaluate the regadenoson-induced blood pressure (BP) response with and without prior caffeine
- To assess the safety and tolerability of regadenoson with and without prior caffeine
- To assess whether the effect of prior caffeine on the MBF response to regadenoson differs between male and female volunteers

### Methodology:

This was a randomized, double-blind, crossover study of regadenoson in normal subjects with and without caffeine. Resting and stress positron emission tomography (PET) scans were performed following regadenoson administration (a single 400 µg intravenous (IV) dose, administered over 10 seconds, followed by a 5 mL saline flush) and following dosing with caffeine 200 mg or placebo on each of 2 study days. 15O water was used as the radionuclide in the PET scans. There was a 1- to 14-day washout period between dosing days. Blood samples and measures of safety were collected until 120 minutes after study drug administration.

### Number of Subjects (Planned and Analyzed):

The study was designed to enroll 52 subjects (26 in each crossover sequence) in order that 40 subjects complete the study with evaluable data. There were 45 subjects enrolled and randomized and 43 subjects dosed with regadenoson of which 41 subjects completed the study, 40 subjects were evaluable for efficacy, and 2 subjects terminated prematurely.

### Diagnosis and Main Criteria for Inclusion:

Healthy adult men or women (≥18 years of age) who provided written informed consent, and who were nonsmokers and regular coffee drinkers (at least one cup per day) were considered for inclusion in the study. Enrolled subjects were to have had no clinically relevant physical findings or electrocardiogram (ECG) findings at baseline. They were also required to abstain from intake of caffeine or other methylxanthines for 24 hours before each study day, and to abstain from all food and beverages except water from 4 hours before the baseline assessments until the final blood sample was taken (5 minutes after the stress PET scan). Female subjects of childbearing potential must have had a negative baseline pregnancy test and have used an acceptable method of birth control for 3 months prior to admission and through 1 week following the study.

Subjects were not eligible for enrollment in the study if they had any illness requiring ongoing treatment. Those with a history of alcohol abuse or drug addiction, or a history of known or suspected bronchoconstrictive and bronchospastic lung disease, or a known allergy to theophylline or aminophylline were not permitted to enroll.

### Test Product, Dose and Mode of Administration, Batch Number:

Open-label study drug was supplied as sterile stock solution in single-use vials each containing 5 mL of regadenoson (0.08 mg/mL). Regadenoson, 400 µg, was administered as a rapid bolus, through an iv catheter over approximately 10 seconds, followed immediately by a 5 mL saline flush. Regadenoson (study drug) had the following CVT lot number: 803604.

### Duration of Treatment:

On each of 2 study days, subjects received a single dose of regadenoson, administered intravenously as a rapid (10-second) bolus of 5 mL, followed by a 5 mL saline flush. There was a 1- to 14-day washout period between doses.

### Reference Therapy, Dose and Mode of Administration, Batch Number:

Caffeine, 200 mg po, or placebo capsule was administered approximately 105 minutes prior to regadenoson. The CVT tracking number for the caffeine capsules was 1341 (Leg 3). These capsules contained caffeine tablets from Bristol-Myers Squibb (NoDoz®) with lot number 405542. The CVT tracking number for the placebo capsules was 1341 (Leg 2).

### Criteria for Evaluation:

### Efficacy:

The primary efficacy measure was the log coronary flow reserve (CFR), which is the ratio of stress MBF after regadenoson dosing to the resting MBF. Plasma caffeine, theophylline, and regadenoson concentrations were measured, and were to be used in exploratory analyses.

### Safety:

Safety measures included adverse events (AEs), serious adverse events, vital signs (HR and BP), ECG, concomitant medications, and a tolerability questionnaire. All available data from subjects who received the single dose of regadenoson were to be included in the statistical summaries.

The primary efficacy analysis was to test whether caffeine reduces CFR after regadenoson administration by at least 10%, using an analysis of variance (ANOVA) with terms for sequence, subject-within-sequence, period, and treatment. The limits of the 95% and 90% confidence intervals (CIs) for the difference of treatment mean values (caffeine-placebo; log scale) were to be exponentiated to obtain CIs for the ratios of the rawscale median values. If the lower limit of this latter 90% CI exceeded 0.9, it could be stated with 95% confidence that prior caffeine administration reduces CFR by less than 10%. The data were also to be analyzed using Wilcoxon's rank-sum test.

The effect of caffeine was to be compared in male and female subjects. Exploratory pharmacodynamic analyses included effect of caffeine on HR and BP and on the relationship between MBF and HR/BP, as well as the correlation between CFR and plasma caffeine concentrations. AEs occurring or worsening after regadenoson administration were to be summarized by severity, relationship to study drug, and prior caffeine status. Vital signs (HR, systolic and diastolic BP, and calculated mean arterial pressure) were to be summarized at individual time points and change-from-baseline values were to be calculated; CIs for the difference in mean values (caffeine-placebo) were to be determined.

Relationships between caffeine and theophylline plasma concentrations and HR and BP were to be explored. ECG intervals and changes from baseline values in ECG intervals were to be presented, as were occurrences of rhythm or conduction abnormalities. Concomitant medication usage was to be summarized.

Tolerability questionnaire responses were to be analyzed using the Wilcoxen rank sum test ("How did you feel?" question) and the exact Cochran-Mantel-Haenszel test (Day 2-only question "How did this test compare to the first test?").

### Efficacy Results:

The log CFR ± SE for the placebo group (n=40) was 1.03 ± 0.06 and log CFR for the caffeine group (n=40) was 0.95 ± 0.06. The CFR (stress/rest) for the placebo group was 2.97 ± 0.16 and for the caffeine group was 2.75 ±0.16.

While there was no change in CFR detected in this study, the study does not rule out nor does it establish a significant interaction between regadenoson and caffeine on log CFR. The exponentiated upper and lower limits of the 95 and 90% confidence intervals for log CFR (caffeine versus placebo difference) are 1.08 and 0.78 and 1.06 and 0.80, respectively.

Since this lower limit is less than 0.9, but the upper limit is > 1, this study cannot establish or rule out an interaction. However, there is 95% confidence that the change in CFR is not ≥ 20%.

There was no significant interaction of caffeine with regadenoson on CFR by sex.

### Safety Results:

AEs occurred at any time in the following classes by percentage of subjects: cardiac disorders 25/43 (58%), respiratory, thoracic and mediastinal disorders 25/43 (58%), nervous system disorders 18/43 (42%), vascular disorders 13/43 (30%), musculoskeletal and connective tissue disorders 12/43 (28%), general disorders and administration site conditions 11/43 (26%), gastrointestinal disorders 2/43 (5%), and ear and labyrinth disorders 1/43 (2%).

The most frequently occurring AEs were dyspnoea 24/43 (56%), palpitations 21/43 (49%), flushing 13/43 (30%), headache 12/43 (28%), sensation of heaviness 12/27 (28%), and paraesthesia 8/43 (19%).

Forty percent (17/43) of subjects had at least one AE with a maximum severity of mild, 49% (21/43) moderate, and 9% (4/43) severe. Ninety-five percent of subjects (41/43) had at least one AE that was considered probably related and 2% (1/43) of patients had at least one AE that was considered possibly related to regadenoson treatment.

Regadenoson-induced headache severity was decreased with caffeine (p = 0.012). There were no reported deaths or SAEs.

Caffeine attenuated the HR increase caused by regadenoson (p < 0.001). There was no effect of caffeine on systolic or diastolic blood pressures in the presence of regadenoson.

After regadenoson dosing, one subject appears to have developed first degree AV block, and one subject appears to have had QTc prolongation (> 500 msec and change of > 60 msec) as determined by ECG analysis that were not reported as AEs.

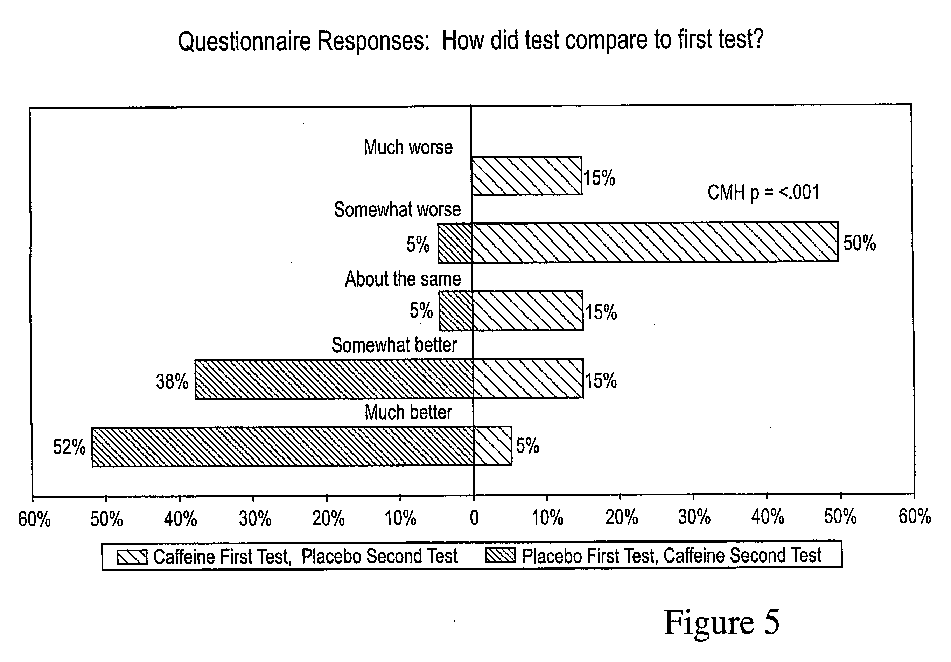

According to the tolerability questionnaire, subjects felt more comfortable during the test with caffeine (p < 0.001), and felt better after the caffeine test than after the placebo test (p < 0.001). Figure 5

While there was no change in CFR detected in this study, the study does not rule out nor does it establish a significant interaction between regadenoson and caffeine on log CFR. The exponentiated upper and lower limits of the 95 and 90% confidence intervals for log CFR (caffeine versus placebo difference) are 1.08 and 0.78 and 1.06 and 0.80, respectively.

Since this lower limit is less than 0.9, but the upper limit is > 1, this study cannot establish or rule out an interaction. However, there is 95% confidence that the change in CFR is not ≥ 20%.

There was no significant interaction of caffeine with regadenoson on CFR by sex.

There was no difference in overall incidence of AEs between the placebo and caffeine groups; however, caffeine attenuated the severity of AEs. Regadenoson-induced headache severity was decreased with caffeine.

## Claims

1. A pharmaceutical composition comprising at least 50 mg caffeine, at least 10 µg of at least one partial A_{2A} receptor agonist, and at least one pharmaceutical excipient, wherein the partial A_{2A} receptor agonist is selected from the group consisting of CVT-3033, Regadenoson, and combinations thereof.

2. The pharmaceutical composition of claim 1, wherein the amount of the partial A_{2A} receptor agonist ranges from about 10 µg to about 600 µg.

3. The pharmaceutical composition of claim 1 or 2, wherein the amount of caffeine ranges from about 50 mg to about 1000 mg, preferably from about 100 mg to about 500 mg, more preferably from about 200 mg to about 400 mg.

4. Caffeine for use in a method of increasing patient tolerability during vasodilator induced myocardial stress perfusion imaging of a mammal, comprising administering a therapeutically effective amount of caffeine and at least 10 µg of at least one partial A_{2A} receptor agonist to the mammal, wherein the partial A_{2A} receptor agonist is selected from the group consisting of CVT-3033, Regadenoson, and combinations thereof.

5. Caffeine for use according to claim 4, wherein the therapeutically effective amount of caffeine is to be administered before the administration of the at least one partial A_{2A} receptor agonist or is to be administered concurrently with the administration of the at least one partial A_{2A} receptor agonist.

6. Caffeine for use according to claim 5, wherein the therapeutically effective amount of caffeine is to be administered no more than 120 minutes, preferably no more than 30 minutes before the administration of the at least one partial A_{2A} receptor agonist.

7. Caffeine for use according to claim 4, wherein the therapeutically effective amount of caffeine and the at least one partial A_{2A} receptor agonist are to be administered as a single pharmaceutical composition.

8. Caffeine for use according to claim 4, wherein the partial A_{2A} receptor agonist is to be administered in an amount ranging from about 10 µg to about 600 µg, preferably in an amount ranging from about 100 µg to about 500 µg.

9. Caffeine for use according to claim 8, wherein the partial A_{2A} receptor agonist is to be administered in a single dose.

10. Caffeine for use according to claim 8, wherein the partial A_{2A} receptor agonist is to be administered by iv bolus.

11. Caffeine for use according to claim 4, wherein the mammal is human.

12. Caffeine for use in a method of increasing patient tolerability during vasodilator induced myocardial stress perfusion imaging of a human, comprising administering from about 50 mg to about 1000 mg caffeine, a radionuclide and a partial A_{2A} receptor agonist in an amount ranging from about 10 µg to about 600 µg wherein the myocardium is to be examined for areas of insufficient blood flow following administration of the radionuclide and the partial A_{2A} receptor agonist, wherein the partial A_{2A} receptor agonist is selected from the group consisting of CVT-3033, Regadenoson, and combinations thereof.

13. Caffeine for use according to claim 12, wherein the radionuclide and the partial A_{2A} receptor agonist are to be administered separately or simultaneously.

14. Caffeine for use according to claim 12, wherein the caffeine and the partial A_{2A} receptor agonist are to be administered separately and preferably the caffeine is to be administered no more than 120 minutes before the administration of the partial A_{2A} receptor agonist.

15. Caffeine for use in a method of increasing patient tolerability during vasodilator induced myocardial stress perfusion imaging of a human, comprising administering from about 50 mg to about 1000 mg, preferably 100 mg to about 500 mg caffeine and from about 10 µg to about 600 µg, preferably about 100 µg to about 500 µg Regadenoson to the human in a single iv bolus.

## Patentansprüche

1. Pharmazeutische Zusammensetzung, die mindestens 50 mg Koffein, mindestens 10 µg mindestens eines teilweisen A_{2A}-Rezeptor-Agonisten und mindestens einen pharmazeutischen Hilfsstoff umfasst, wobei der teilweise A_{2A}-Rezeptor-Agonist ausgewählt ist aus der Gruppe, bestehend aus CVT-3033, Regadenoson und Kombinationen davon.

2. Pharmazeutische Zusammensetzung nach Anspruch 1, wobei die Menge des teilweisen A_{2A}-Rezeptor-Agonisten von etwa 10 µg bis etwa 600 µg reicht.

3. Pharmazeutische Zusammensetzung nach Anspruch 1 oder 2, wobei die Menge von Koffein von etwa 50 mg bis etwa 1000 mg, vorzugsweise von etwa 100 mg bis etwa 500 mg, mehr bevorzugt von etwa 200 mg bis etwa 400 mg reicht.

4. Koffein für eine Verwendung in einem Verfahren zur Erhöhung der Patientenerträglichkeit während Vasodilator-induzierter Myokardperfusionsdarstellung unter Belastung eines Säugers, das eine Verabreichung einer therapeutisch wirksamen Menge von Koffein und von mindestens 10 µg mindestens eines teilweisen A_{2A}-Rezeptor-Agonisten an den Säuger umfasst, wobei der teilweise A_{2A}-Rezeptor-Agonist ausgewählt ist aus der Gruppe, bestehend aus CVT-3033, Regadenoson und Kombinationen davon.

5. Koffein für eine Verwendung gemäß Anspruch 4, wobei die therapeutisch wirksame Menge von Koffein vor der Verabreichung des mindestens einen teilweisen A_{2A}-Rezeptor-Agonisten zu verabreichen ist oder gleichzeitig mit der Verabreichung des mindestens einen teilweisen A_{2A}-Rezeptor-Agonisten zu verabreichen ist.

6. Koffein für eine Verwendung gemäß Anspruch 5, wobei die therapeutisch wirksame Menge von Koffein nicht mehr als 120 Minuten, vorzugsweise nicht mehr als 30 Minuten vor der Verabreichung des mindestens einen teilweisen A_{2A}-Rezeptor-Agonisten zu verabreichen ist.

7. Koffein für eine Verwendung gemäß Anspruch 4, wobei die therapeutisch wirksame Menge von Koffein und des mindestens einen teilweisen A_{2A}-Rezeptor-Agonisten als eine einzige pharmazeutische Zusammensetzung zu verabreichen sind.

8. Koffein für eine Verwendung gemäß Anspruch 4, wobei der teilweise A_{2A}-Rezeptor-Agonist in einer Menge, die von etwa 10 µg bis etwa 600 µg reicht, vorzugsweise in einer Menge, die von etwa 100 µg bis etwa 500 µg reicht, zu verabreichen ist.

9. Koffein für eine Verwendung gemäß Anspruch 8, wobei der teilweise A_{2A}-Rezeptor-Agonist in einer einzigen Dosis zu verabreichen ist.

10. Koffein für eine Verwendung gemäß Anspruch 8, wobei der teilweise A_{2A}-Rezeptor-Agonist durch iv-Bolus zu verabreichen ist.

11. Koffein für eine Verwendung gemäß Anspruch 4, wobei der Säuger ein Mensch ist.

12. Koffein für eine Verwendung in einem Verfahren zur Erhöhung der Patientenerträglichkeit während Vasodilator-induzierter Myokardperfusionsdarstellung unter Belastung eines Menschen, das eine Verabreichung von etwa 50 mg bis etwa 1000 mg Koffein, eines Radionuklids und eines teilweisen A_{2A}-Rezeptor-Agonisten in einer Menge, die von etwa 10 µg bis etwa 600 µg reicht, umfasst, wobei das Myokard auf Bereiche eines unzureichenden Blutflusses nach Verabreichung des Radionuklids und des teilweisen A_{2A}-Rezeptor-Agonisten zu untersuchen ist, wobei der teilweise A_{2A}-Rezeptor-Agonist ausgewählt ist aus der Gruppe, bestehend aus CVT-3033, Regadenoson und Kombinationen davon.

13. Koffein für eine Verwendung gemäß Anspruch 12, wobei das Radionuklid und der teilweise A_{2A}-Rezeptor-Agonist getrennt oder gleichzeitig zu verabreichen sind.

14. Koffein für eine Verwendung gemäß Anspruch 12, wobei das Koffein und der teilweise A_{2A}-Rezeptor-Agonist getrennt zu verabreichen sind und vorzugsweise das Koffein nicht mehr als 120 Minuten vor der Verabreichung des teilweisen A_{2A}-Rezeptor-Agonisten zu verabreichen ist.

15. Koffein für eine Verwendung in einem Verfahren zur Erhöhung der Patientenerträglichkeit während Vasodilator-induzierter Myokardperfusionsdarstellung unter Belastung eines Menschen, das eine Verabreichung von etwa 50 mg bis etwa 1000 mg, vorzugsweise 100 mg bis etwa 500 mg Koffein und von etwa 10 µg bis etwa 600 µg, vorzugsweise etwa 100 µg bis etwa 500 µg Regadenoson an den Menschen in einem einzigen iv-Bolus umfasst.

## Revendications

1. Composition pharmaceutique comprenant au moins 50 mg de caféine, au moins 10 µg d'au moins un agoniste du récepteur A_{2A} partiel, et au moins un excipient pharmaceutique, tandis que l'agoniste du récepteur A_{2A} partiel, est choisi dans le groupe consistant en CVT-3033, Regadenoson, et leurs combinaisons.

2. Composition pharmaceutique selon la revendication 1, dans laquelle la quantité de l'agoniste du récepteur A_{2A} partiel se situe entre environ 10 µg et environ 600 µg.

3. Composition pharmaceutique selon la revendication 1 ou 2, dans laquelle la quantité de caféine se situe entre environ 50 mg et environ 1000 mg, de préférence entre environ 100 mg et environ 500 mg, plus préférablement entre environ 200 mg et environ 400 mg.

4. Caféine pour utilisation dans un procédé pour augmenter la tolérance d'un patient pendant une imagerie de perfusion au cours de l'épreuve d'effort myocardique induite par un vasodilatateur chez un mammifère, comprenant l'administration d'une quantité thérapeutiquement efficace de caféine et d'au moins 10 µg d'au moins un agoniste du récepteur A_{2A} partiel au mammifère, tandis que l'agoniste du récepteur A_{2A} partiel est choisi dans le groupe consistant en CVT-3033, Regadenoson, et leurs combinaisons.

5. Caféine pour utilisation selon la revendication 4, dans laquelle la quantité thérapeutiquement efficace de caféine est destinée à être administrée avant l'administration du au moins un agoniste du récepteur A_{2A} partiel ou est destinée à être administrée concurremment avec l'administration du au moins un agoniste du récepteur A_{2A} partiel.

6. Caféine pour utilisation selon la revendication 5, dans laquelle la quantité thérapeutiquement efficace de caféine est destinée à être administrée pas plus de 120 minutes, de préférence pas plus de 30 minutes avant l'administration du au moins un agoniste du récepteur A_{2A} partiel.

7. Caféine pour utilisation selon la revendication 4, dans laquelle la quantité thérapeutiquement efficace de caféine et le au moins un agoniste du récepteur A_{2A} partiel sont destinés à être administrés sous la forme d'une composition pharmaceutique unique.

8. Caféine pour utilisation selon la revendication 4, dans laquelle l'agoniste du récepteur A_{2A} partiel est destiné à être administré en une quantité se situant entre environ 10 µg et environ 600 µg, de préférence en une quantité se situant entre environ 100 µg et environ 500 µg.

9. Caféine pour utilisation selon la revendication 8, dans laquelle l'agoniste du récepteur A_{2A} partiel est destiné à être administré en une dose unique.

10. Caféine pour utilisation selon la revendication 8, dans laquelle l'agoniste du récepteur A_{2A} partiel est destiné à être administré par un embol iv.

11. Caféine pour utilisation selon la revendication 4, dans laquelle le mammifère est un humain.

12. Caféine pour utilisation dans un procédé pour augmenter la tolérance d'un patient pendant une imagerie de perfusion sous stress myocardique induit par un vasodilatateur chez un humain, comprenant l'administration de 50 mg à environ 1000 mg de caféine, d'un radionucléide et d'un agoniste du récepteur A_{2A} partiel en une quantité se situant entre environ 10 µg et environ 600 µg, tandis que le myocarde est destiné à être examiné pour les aires de circulation sanguine insuffisante après administration du radionucléide et de l'agoniste du récepteur A_{2A} partiel, tandis que l'agoniste du récepteur A_{2A} partiel est choisi dans le groupe consistant en CVT-3033, Regadenoson, et leurs combinaisons.

13. Caféine pour utilisation selon la revendication 12, dans laquelle le radionucléide et l'agoniste du récepteur A_{2A} partiel sont destinés à être administrés séparément ou simultanément.

14. Caféine pour utilisation selon la revendication 12, dans laquelle la caféine et l'agoniste du récepteur A_{2A} partiel sont destinés à être administrés séparément et de préférence la caféine est destinée à être administrée pas plus de 120 minutes avant l'administration de l'agoniste du récepteur A_{2A} partiel.

15. Caféine pour utilisation dans un procédé pour augmenter la tolérance d'un patient pendant une imagerie de perfusion sous effort myocardique induit par un vasodilatateur chez un humain, comprenant l'administration d'environ 50 mg à environ 1000 mg, de préférence 100 mg à environ 500 mg de caféine et d'environ 10 µg à environ 600 µg, de préférence d'environ 100 µg à environ 500 µg de Regadenoson à l'humain en un embol iv unique.
